# FASCICULE DE BREVET EUROPEEN

(11) **EP 1 345 579 B1**
(45) Date de publication et mention de la délivrance du brevet: **29.06.2005**
(21) Numéro de dépôt: 01272052.0
(22) Date de dépôt: 26.11.2001
(51) Int. Cl.: A61K 7/13

(54) **COMPOSITION POUR LA TEINTURE D'OXYDATION DES FIBRES KERATINIQUES COMPRENANT UN AMINO PYRAZOLE ET UN COMPOSE MINERAL**
OXIDATIONSHAARFÄRBEMITTEL ENTHALTEND EIN AMINOPYRAZOL UND EINE ANORGANISCHE VERBINDUNG
COMPOSITION FOR OXIDATION DYEING OF KERATINOUS FIBRES COMPRISING AN AMINO PYRAZOLE AND A MINERAL COMPOUND

(30) Priorité: 22.12.2000 FR 0016956
(43) Date de publication de la demande: 24.09.2003
(73) Titulaire: L'OREAL, 75008 Paris (FR)
(72) Inventeur: COTTERET, Jean, F-78480 Verneuil-sur-Seine (FR)
(74) Mandataire: Fevrier, Murielle
(86) Numéro de dépôt international: PCT/FR2001/003726
(87) Numéro de publication internationale: WO 2002/051371

(56) Documents cités:
- EP-A- 0 749 748
- WO-A-98/22078
- DE-A- 3 843 892
- DE-A- 4 208 297
- DE-A- 4 234 886
- DE-A- 19 543 988
- DE-A- 19 951 010

## Description

La présente invention a pour objet une composition pour la teinture d'oxydation des fibres kératiniques, en particulier des fibres kératiniques humaines telles que les cheveux, comprenant au moins une base d'oxydation choisie parmi les 4,5 ou 3,4- diamino pyrazoles et les triamino pyrazoles, en association avec au moins un composé minéral sélectionné, ainsi que le procédé de teinture mettant en oeuvre cette composition avec un agent oxydant.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols ou encore des composés hétérocycliques tels que des dérivés de pyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec les bases d'oxydation en les associant à des coupleurs ou modificateurs de coloration convenablement choisis, ces derniers pouvant être choisis notamment parmi des métadiamines aromatiques, des métaaminophénols, des métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine.

Il a déjà été proposé, notamment dans les demandes de brevet allemand DE 3 843 892, DE 4 234 887, DE 4 234 886, DE 4 234 885 ou DE 195 43 988 des compositions pour la teinture d'oxydation des fibres kératiniques contenant à titre de base d'oxydation des dérivés de pyrazole tels que des 4,5-diamino pyrazoles, des 3,4-diamino pyrazoles ou des 3,4,5-triamino pyrazoles. De telles compositions ne sont cependant pas entièrement satisfaisantes car lors des processus de coloration se produisent des réactions secondaires qui peuvent avoir des effets négatifs du point de vue de l'innocuité et des propriétés des colorations obtenues et notamment de la puissance et de la ténacité des colorations vis à vis des diverses agressions que peuvent subir les cheveux.

On connaît également dans la demande WO01/34104 des compositions pour la teinture d'oxydation des fibres kératiniques comprenant l'association d'une base d'oxydation du type 4,5-diaminopyrazole, d'un sel de zinc et d'un dérivé de 1,4-diazacycloheptane ou d'un de ses sels de formule suivante : 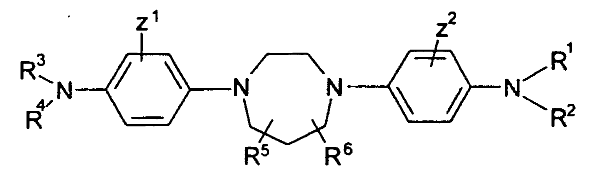 où R¹, R², R³ et R⁴ , identiques ou différents, désignent un hydrogène, un alkyle ou un hydroxyalkyle en C₁-C₄ ou bien un dihydroxyalkyle en C₂-C₄ ;
Z¹ et Z², identiques ou différents, désignent hydrogène, chlore, fluor, un alkyle en C₁-C₄, un hydroxyalkyle en C₁-C₄, un aminoalkyle en C₁-C₄, un alcoxy en C₁-C₄ ou un dihydroxyalkyle en C₂-C₄ ou un groupe allyle ;
R⁵ et R⁶ , identiques ou différents, désignent un hydrogène, un alkyle en C₁-C₄.

L'invention a pour but de développer de nouvelles compositions tinctoriales ne présentant pas les inconvénients des teintures de la technique antérieure, en particulier, des teintures puissantes, particulièrement résistantes aux diverses agressions que peuvent subir les cheveux et présentant une bonne innocuité.

A cet effet, l'invention a pour objet une composition pour la teinture d'oxydation des fibres kératiniques humaines et en particulier des fibres kératiniques humaines telles que les cheveux, comprenant, dans un milieu approprié pour la teinture :
- au moins une base d'oxydation choisie parmi les 4,5 ou 3,4-diamino pyrazoles et les triamino pyrazoles, et
- au moins un composé minéral choisi parmi les silices, les oxydes ou hydroxydes d'aluminium, les hydrosilicates d'aluminium, de magnésium, de fer seul ou en mélange, et les sels de zinc ;
ladite composition ne contenant pas l'association d'une base d'oxydation du type 4,5- diaminopyrazole, d'un sel de zinc et d'un dérivé de 1,4-diazacycloheptane ou d'un de ses sels de formule suivante : 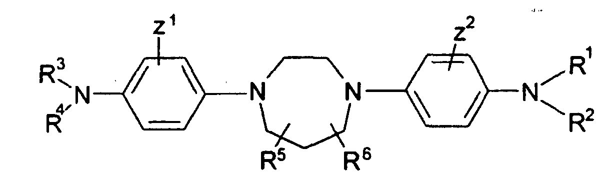 où R¹, R², R³ et R⁴ , identiques ou différents, désignent un hydrogène, un alkyle ou un hydroxyalkyle en C₁-C₄ ou bien un dihydroxyalkyle en C₂-C₄ ;
Z¹ et Z², identiques ou différents, désignent hydrogène, chlore, fluor, un alkyle en C₁-C₄, un hydroxyalkyle en C₁-C₄, un aminoalkyle en C₁-C₄, un alcoxy en C₁-C₄ ou un dihydroxyalkyle en C₂-C₄ ou un groupe allyle ;
R⁵ et R⁶ , identiques ou différents, désignent un hydrogène, un alkyle en C₁-C₄.

La composition de teinture d'oxydation de l'invention permet d'obtenir avec une bonne innocuité des colorations puissantes aux nuances variées, peu sélectives et présentant d'excellentes propriétés de résistance à la fois vis à vis des agents atmosphériques tels que la lumière et les intempéries et vis à vis de la transpiration et des différents traitements que peuvent subir les cheveux (shampooings, déformations permanentes).

Sauf indication contraire, tous les radicaux, substituants, groupements chaines dans le cadre de l'invention sont linéaires ou ramifiés, substitués ou non.
Parmi les silices utiles dans la composition de l'invention, on peut citer les silices cristallines, microcristallines, et non cristallines

A titre d'exemple, on peut citer comme silices cristallines le quartz, la tridymite, la cristobalite, la kéatite, la coesite et la stishovite. Les silices microcristallines sont par exemple la diatomite.

Parmi les formes non cristallines, on peut utiliser la silice vitreuse et d'autres types de silices amorphes telles que les silices colloidales, les silicagels, les silices précipités et les silices fumées comme les aérosils et les silices pyrogéniques.

Parmi les hydroxydes d'aluminium utilisables selon l'invention, on peut citer les composés de composition Al(OH)₃ tels que le trihydrate d'α-alumine hydrargillite ou gibbsite ou le trihydrate de β-alumine bayerite et les composés de composition AlOOH tels que le nouveau β-trihydrate nordstrandite, le monohydrate d'α-alumine boehmite et le monohydrate de β-alumine diaspore.

Parmi les oxydes d'aluminium utilisables selon l'invention, on peut les alumines activées dont la structure cristalline est celle des alumines de transition γ, η, χ ou ρ, les alumines calcinées comme l'alumine α. On peut aussi citer les alumines β tels que les sodium β-aluminates, les sodium γ-aluminates, les sodium β-alumines, les potassium β-alumines, les magnésium β-alumines, les calcium β-alumines, les strontium β-alumines, les baryum β-atumines, les litium ξ-alumines.

Parmi les hydrosilicates d'aluminium , on peut citer la kaolinite, la dicktite, la nacrite, l'halloysite-endellite que l'on retrouve dans les argiles de type kaolins, ainsi que la pyrophyllite et la beidellite que l'on retrouve dans les smectites.,

Parmi les hydrosilicates de magnésium , on peut citer le talc que l'on retrouve dans les smectites et les serpentines telle que le chrysotile

Parmi les hydrosilicates contenant plusieurs des éléments métalliques ci dessus, on peut citer la montmorillonite et la saponite (Aluminium+magnésium), la nontronite (Aluminium+fer), la sauconite (Aluminium+magnésium+fer) que l'on retrouve dans les smectites, l'amésite (Aluminium+magnésium), la chamosite et l'illite (fer+aluminium+magnésium).

Parmi les sels de zinc, on peut citer les sels obtenus à partir d'un acide minéral comme le sulfate , le chlorhydrate, le nitrate ,le carbonate, l'hydrogénocarbonate, l'ortho phosphate, l'ortho dihydrogénophosphate, l'ortho dihydrogénophosphate et les sels obtenus à partir d'acides organiques comme le tartrate, le citrate, l'acétate ou le lactate.

Le ou les composés minéraux représentent de préférence de 0,00001 à 10 % en poids environ du poids total de la composition tinctoriale, encore plus préférentiellement de 0,001 à 5 % et encore plus préférentiellement de 0,001 à 3 % en poids environ de ce poids.

Parmi les 4,5 ou 3,4-diamino pyrazoles utiles dans les compositions tinctoriales de l'invention, on peut particulièrement citer les diamino pyrazoles choisis parmi les 4,5 ou 3,4-diamino pyrazoles de formules (I) ou (II) suivantes et/ou leurs sels d'addition avec un acide : 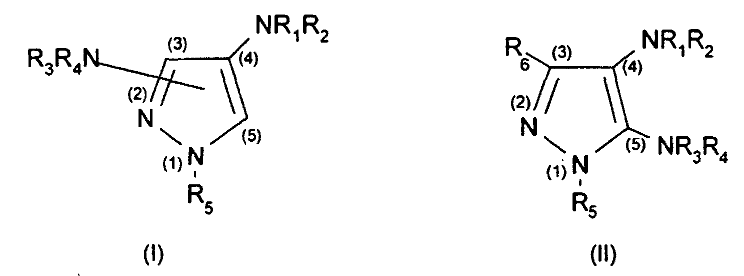 dans laquelle :
- R₁, R₂, R₃, R₄ et R₅, identiques ou différents, représentent un atome d'hydrogène ; un radical alkyle en C₁-C₆ non substitué ou substitué par au moins un substituant choisi parmi OR, NHR, NRR', SR, SOR, SO₂R, COR, COOH, CONH₂, CONHR, CONRR', PO(OH)₂, SH, SO₃X, un hétérocycle non cationique, Cl, Br ou I, X désignant un atome d'hydrogène, Na, K, ou NH₄, et R et R', identique ou différents, représentant un alkyle ou alcényle en C₁-C₄ ; un radical hydroxyalkyle en C₂-C₄ ; un radical aminoalkyle en C₂-C₄ ; un radical phényle ; un radical phényle substitué par un atome d'halogène ou un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, méthylènedioxy ou amino ; un radical 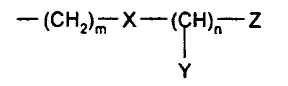
dans lequel m et n sont des nombres entiers, identiques ou différents, compris entre 0 et 3 inclusivement, X représente un atome d'oxygène ou bien le groupement NH, Y représente un atome d'hydrogène ou bien un radical alkyle en C₁-C₄; et Z représente un radical méthyle lorsque n est égal à 0, ou Z représente un radical alkyle en C₁-C₄, un groupement OR ou NR"R"' lorsque n est supérieur ou égal à 1, R" et R"', identiques ou différent, désignant un atome d'hydrogène, un radical alkyle en C₁-C₄ ; ou R₅ forme avec l'atome d'azote du groupement NR₃R₄ en position 5, un hétérocycle comprenant au moins 4 chaînons,
- R₆ représente un radical alkyle en C₁-C₆ ; un radical hydroxyalkyle en C₁-C₄ ; un radical aminoalkyle en C₁-C₄ ; un radical alkyle(C₁-C₄)aminoalkyle en C₁-C₄ ; un radical dialkyle (C₁-C₄)-aminoalkyle en C₁-C₄ ; un radical hydroxyalkyle (C₁-C₄)-aminoalkyle en C₁-C₄ ; un radical alcoxy(C₁-C₄)méthyle ; un radical phényle ; un radical phényle substitué par un atome d'halogène ou par un radical alkyle(C₁-C₄), alcoxy(C₁-C₄), nitro, trifluorométhyle, amino ou alkyl(C₁-C₄)amino ; un radical benzyle ; un radical benzyle substitué par un atome d'halogène ou par un radical alkyle(C₁-C₄), alcoxy(C₁-C₄), nitro, trifluorométhyle, amino ou alkyl(C₁-C₄)amino; un hétérocycle choisi parmi le thiophène, le furane et la pyridine, ou encore un radical -(CH₂)ₚ-O-(CH₂)_{q}-OR", dans lequel p et q sont des nombres entiers, identiques ou différents, compris entre 1 et 3 inclusivement et R" est tel que défini précédemment,
étant entendu que :
- au moins un des radicaux R₁, R₂, R₃ et R₄ représente un atome d'hydrogène.

Parmi les triamino pyrazoles utiles à titre de base d'oxydation dans les compositions tinctoriales conformes à l'invention, on peut plus particulièrement citer les composés de formule (III) suivante, et leurs sels d'addition avec un acide : 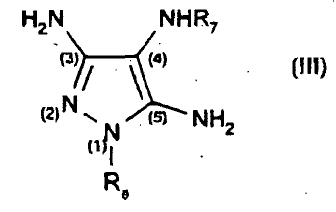 dans laquelle :
- R₇ et R₈, identiques ou différents, représentent un atome d'hydrogène, un radical alkyle en C₁-C₄ ou hydroxyalkyle en C₂-C₄.

Parmi les 4,5 ou 3,4-diamino pyrazoles de formule (I) ci-dessus, on peut plus particulièrement citer le 4,5-diamino 1-(4'-méthoxybenzyle) pyrazole, le 4,5-diamino 1-(4'-méthylbenzyle) pyrazole, le 4,5-diamino 1-(4'-chlorobenzyle) pyrazole, le 4,5-diamino 1-(3'-méthoxybenzyle) pyrazole, la 4-amino 1-(4'-méthoxybenzyle) 5-méthylamino pyrazole, la 4-amino 5-(β-hydroxyéthyl)amino 1-(4'-méthoxybenzyle) pyrazole, le 4-amino 5-(β-hydroxyéthyl)amino 1-méthyl pyrazole, le 4-amino (3) 5-méthylamino pyrazole, le 3-(5),4-diamino pyrazole, le 4,5-diamino 1-méthyl pyrazole, le 4,5-diamino 1-éthyl pyrazole, le 4,5-diamino 1-isopropyl pyrazole,le 4,5-diamino 1-hydroxyéthyl pyrazole, le 4,5-diamino 1-benzyl pyrazole, le 4-amino 5-hydroxyéthylamino 1-hydroxyéthyl pyrazole, le 4-diamino 5-méthylamino 1-hydroxyéthyl pyrazole, la 3-amino 4,5,7,8-tétrahydro pyrazolo [1,5-a] pyrimidine, le 7-amino 2,3-dihydro 1-H-imidazol [1,2-b] pyrazole, la 3-amino 8-méthyl 4,5,7,8-tétrahydropyrazolo [1,5-a] pyrimidine, l'acide 2-(4,5-diamino-pyrazol-1-yl)- 1-éthane sulfonique, le 2(4,5-diamino-pyrazol-1-yl)-acétamide, l'acide 2-( 4,5-diamino-pyrazol-1-yl)-acélique, la 2-(2-diméthylamino-éthyl)-2H-pyrazole-3,4-diamine, la 2-(2-méthoxy-éthyl)-2H-pyrazole-3,4-diamine et leurs sels d'addition avec un acide.

Les diaminopyrazoles utiles dans la présente invention peuvent être obtenus par des procédés de synthèses bien connus de l'homme du métier. Par exemple, les 4,5-diamino pyrazoles de formule (II) peuvent être préparés selon le procédé de synthèse tel que décrit par exemple dans la demande de brevet français FR-A-2 733 749.

Parmi les 4,5-diamino pyrazoles de formule (II) ci-dessus, on peut plus particulièrement citer :
- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino 1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino 3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphenyl) pyrazole,
- le 4,5-diamino 3-méthyl 1-(3'-méthoxyphényl) pyrazole,
- le 4,5-diamino 3-méthyl 1-(4'-méthoxyphényl) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminométhyl 1-méthyl pyrazole,
- le 4,5-diamino 3-diméthylaminométhyl 1-éthyl pyrazole,
- le 4,5-diamino 3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,
- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phényl 3-méthyl pyrazole,
- le 4-amino 5-(β-hydroxyéthyl)amino 1-(2-méthoxyphényl) 3-méthyl pyrazole,
- le 4-amino 5-(β-hydroxyéthyl)amino 1-(3-méthoxyphényl) 3-méthyl pyrazole,
- le 4-amino 5-(β-hydroxyéthyl)amino 1-(4-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 4,5-diamino 1,3-diméthyl pyrazole,
- le 4,5-diamino 3-tert.butyl 1-méthyl pyrazole,
- le 4,5-diamino 1-tert.butyl 3-méthyl pyrazole,
- le 4,5-diamino 1-méthyl 3-phényl pyrazole,
- le 4,5-diamino 1-(β-hydroxyéthyl) 3-méthyl pyrazole,
- le 4,5-diamino 1-(β-hydroxyéthyl) 3-phényl pyrazole,
- le 4,5-diamino 1-méthyl 3-(2'-chlorophényl) pyrazole,
- le 4,5-diamino 1-méthyl 3-(4'-chlorophényl) pyrazole,
- le 4,5-diamino 1-méthyl 3-(3'-trifluorométhylphényl) pyrazole,
- le 4,5-diamino 1,3-diphényl pyrazole,
- le 4,5-diamino 3-méthyl 1-phényl pyrazole,
- le 4-amino 1,3-diméthyl 5-phénylamino pyrazole,
- le 4-amino 1-éthyl 3-méthyl 5-phénylamino pyrazole,
- le 4-amino 1,3-diméthyl 5-méthylamino pyrazole,
- le 4-amino 3-méthyl 1-isopropyl 5-méthylamino pyrazole,
- le 4-amino 3-isobutoxyméthyl 1-méthyl 5-méthylamino pyrazole,
- le 4-amino 3-méthoxyéthoxyméthyl 1-méthyl 5-méthylamino pyrazole,
- le 4-amino 3-hydroxyméthyl 1-méthyl 5-méthylamino pyrazole,
- le 4-amino 1,3-diphényl 5-phénylamino pyrazole,
- le 4-amino 3-méthyl 5-méthylamino 1-phényl pyrazole,
- le 4-amino 1,3-diméthyl 5-hydrazino pyrazole,
- le 5-amino 3-méthyl 4-méthylamino 1-phényl pyrazole,
- le 5-amino 1-méthyl 4-(N,N-méthylphényl)amino 3-(4'-chlorophényl) pyrazole,
- le 5-amino 3-éthyl 1-méthyl 4-(N,N-méthylphényl)amino pyrazole,
- le 5-amino 1-méthyl 4-(N,N-méthylphényl)amino 3-phényl pyrazole,
- le 5-amino 3-éthyl 4-(N,N-méthylphényl)amino pyrazole,
- le 5-amino 4-(N,N-méthylphényl)amino 3-phényl pyrazole,
- le 5-amino 4-(N,N-méthylphényl)amino 3-(4'-méthylphényl) pyrazole,
- le 5-amino 3-(4'-chlorophényl) 4-(N,N-méthylphényl)amino pyrazole,
- le 5-amino 3-(4'-méthoxyphényl) 4-(N,N-méthylphényl)amino pyrazole,
- le 4-amino 5-méthylamino 3-phényl pyrazole,
- le 4-amino 5-éthylamino 3-phényl pyrazole,
- le 4-amino 5-éthylamino 3-(4'-méthylphényl) pyrazole,
- le 4-amino 3-phényl 5-propylamino pyrazole.
- le 4-amino 5-butylamino 3-phényl pyrazole,
- le 4-amino 3-phényl 5-phénylamino pyrazole,
- le 4-amino 5-benzylamino 3-phényl pyrazole,
- le 4-amino 5-(4'-chlorophényl)amino 3-phényl pyrazole,
- le 4-amino 3-(4'-chlorophényl) 5-phénylamino pyrazole,
- le 4-amino 3-(4'-méthoxyphényl) 5-phénylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoéthyl)amino 1,3-diméthyl pyrazole,
et leurs sels d'addition avec un acide.

Parmi les 4,5 ou 3,4-diamino pyrazoles de formule (I) ci-dessus, on préfère plus particulièrement :
- le 4,5-diamino 1- benzyle pyrazole
- le 4,5-diamino 1-(4'-chlorobenzyle) pyrazole
- le 4,5-diamino 1-méthyl pyrazole
- le 4,5-diamino 1-hydroxyéthyl pyrazole
- la 2-(2-méthoxy-éthyl)-2H-pyrazole-3,4-diamine
et leurs sels d'addition avec un acide.

Parmi les 4,5-diamino pyrazoles de formule (II) ci-dessus, on préfère plus particulièrement :
- le 4,5-diamino 1,3-diméthyl pyrazole,
- le 4,5-diamino 3-méthyl 1-phényl pyrazole,
- le 4,5-diamino 1-méthyl 3-phényl pyrazole,
- le 4-amino 1,3-diméthyl 5-hydrazino pyrazole,
- le 1-benzyl 4,5-diamino 3-méthyl pyrazole,
- le 4,5-diamino 3-tert-butyl 1-méthyl pyrazole,
- le 4,5-diamino 1-tert-butyl 3-méthyl pyrazole,
- le 4,5-diamino 1-(β-hydroxyéthyl) 3-méthyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 1-éthyl 3-hydroxyméthyl pyrazole,
- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,
et leurs sels d'addition avec un acide.
Parmi les triamino pyrazoles de formule (III) ci-dessus, on peut plus particulièrement citer le 3,4,5-triamino pyrazole, le 1-méthyl 3,4,5-triamino pyrazole, le 3,5-diamino, 1-méthyl 4-méthylamino pyrazole, et le 3,5-diamino 4-(β-hydroxyéthyl)amino 1-méthyl pyrazole, et leurs sels d'addition avec un acide.

Le ou les 4,5 ou 3,4-diamino pyrazoles et/ou le ou les triamino pyrazoles conformes à l'invention et/ou le ou les sels d'addition avec un acide correspondants représentent de préférence de 0,0005 à 12 % en poids environ du poids total de la composition tinctoriale, et plus préférentiellement de 0,005 à 6 % en poids environ de ce poids.

De préférence, le rapport pondéral du ou des composés minéraux avec le ou les 4,5 ou 3,4-diamino pyrazoles et/ou le ou les triamino pyrazoles et/ou le ou les sels d'addition avec un acide est compris entre 0,001 et 100 et encore plus préférentiellement entre 0,01 et 10.

Les compositions tinctoriales conformes à l'invention contiennent de préférence au moins un coupleur. Les coupleurs utilisables sont ceux classiquement utilisés pour la teinture d'oxydation et notamment les métaphénylènediamines, les métaaminophénols, les métadiphénols, les dérivés de naphtol et les coupleurs hétérocycliques.

Les méta-phénylènediamines, les méta-aminophénols et les méta-diphénols pouvant être utilisés à titre de coupleurs additionnels dans la composition tinctoriale conforme à l'invention sont de préférence choisis parmi les composés répondant à la formule (1) suivante et leurs sels d'addition avec un acide : 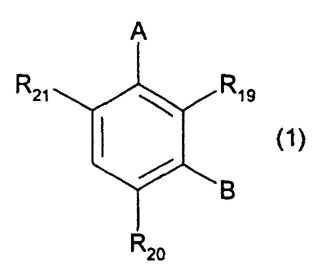 dans laquelle :
- A et B, identiques ou différents, représentent un radical hydroxyle, amino ou -NHR₂₂ dans lequel R₂₂ représente un radical alkyle en C₁-C₄, monohydroxyalkyle en C₁-C₄, ou polyhydroxyalkyle en C₂-C₄,
- R₁₉, R₂₀ et R₂₁, identiques ou différents, représentent un atome d'hydrogène ou d'halogène tel qu'un atome de brome, de chlore, d'iode ou de fluor, un radical alkyle en C₁-C₄, alcoxy en C₁-C₄, monohydroxyalkyle en C₁-C₄ ou polyhydroxyalkyle en C₂-C₄, monohydroxyalcoxy en C₁-C₄ ou polyhydroxyalcoxy en C₂-C₄.

Parmi les composés de formule (1) ci-dessus, on peut notamment citer le 2-méthyl 5-amino phénol, le 2-méthyl 5-amino 6-chloro phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 2,6bis(β-hydroxyéthylamino) toluène et leurs sels d'addition avec un acide.

Le ou les coupleurs hétérocycliques pouvant être utilisés à titre de coupleurs additionnels dans la composition tinctoriale conforme à l'invention peuvent notamment être choisis parmi les dérivés indoliques, les dérivés indoliniques, les dérivés pyridiniques, les dérivés pyrimidiniques, les pyrazolones, et leurs sels d'addition avec un acide.

Parmi ces coupleurs hétérocycliques, on peut en particulier citer à titre d'exemple, le sésamol, le 1-N(β-hydroxyéthyl)amino 3'4-méthylènedioxy benzène,le 6-hydroxy indole, le 4-hydroxy indole, le 4-hydroxy N-méthyl indole, la 6-hydroxy indoline, la 6-hydroxybenzomorpholine, la 2,6-dihydroxy 4-méthyl pyridine, la 3,5-diamino 2,6-diméthoxy pyridine, la 2-amino 3-hydroxy pyridine le 1-H 3-méthyl pyrazole 5-one, le 1-phényl 3-méthyl pyrazole 5-one, et leurs sels d'addition avec un acide.
Parmi les dérivés de naphtol on peut citer l'α-naphtol, le 2-méthyl-1-naphtol.

Le ou les coupleurs additionnels représentent de préférence de 0,0001 à 10 % en poids environ du poids total de la composition tinctoriale et encore plus préférentiellement de 0,005 à 5 % en poids environ de ce poids.

Les compositions tinctoriales conformes à l'invention peuvent également contenir d'autres bases d'oxydation classiquement utilisées pour la teinture d'oxydation, différentes d'un diamino pyrazole et d'un triamino pyrazole et/ou des colorants directs notamment pour modifier les nuances ou les enrichir en reflets.

Les bases d'oxydation additionnelles utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques autres que les pyrazoles de l'invention ainsi que leurs sels d'addition avec un acide, et notamment;
- **(I)** les paraphénylènediamines de formule (2) suivante et leurs sels d'addition avec un acide : 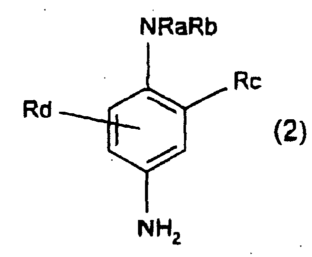 dans laquelle :
   **R**ₐ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   **R**_{b} représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté;
   **R**_{c} représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acélylaminoalcoxy en C₁-C₄, mésylaminoalcoxy en C₁-C₄ ou carbamoylaminoalcoxy en C₁-C₄.
   **R**_{d} représente un atome d'hydrogène, d'halogène ou un radical alkyle en C₁-C₄.
   Rₐ et R_{b} peuvent également former avec l'atome d'ozote qui les porte un hétérocycle azoté à 5 ou 6 chaînons éventuellement substitué par un ou plusieurs groupements alkyle, hydroxy ou uréido.

   Parmi les groupements azotés de la formule (2) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (2) ci-dessus, on peut plus particulièrement citer la paraphénylènediamine, la paratoluylènediamine, la 2-chloroparaphénylènediamine, la 2,3-diméthyl-paraphénylènediamine, la 2,6-diméthylparaphénylènediamine, la 2,6-diéthyl-paraphénylènediamine, la 2,5-diméthylparaphénylènediamine, la N,N-diméthyl-paraphénylènediamine, la N,N-diéthylparaphénylènediaminé, la N,N-dipropyl-paraphénylènediamine, la 4-amino-N,N-diéthyl-3-méthyl-aniline, la N,N-bis-(β-hydroxyéthyl)-paraphénylènediamine, la 4-amino-N,N-bis-(β-hydroxyéthyl)-3-méthyl-aniline, la 4-amino-3-chloro-N,N-bis-(β-hydroxyéthyl)-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N, N-diméthyl-3-méthylparaphénylènediamine, la N,N-(éthyl, β-hydroxyéthyl)-paraphénylènediamine, la N-(β,γ-dihydroxypropyl)paraphénylènediamine, la N-(4'-aminophényl)-paraphénylènediamine, la N-phényl-paraphénylènediamine, la 2-β-hydroxyéthyloxy-paraphénylènediamine, et leurs sels d'addition avec un acide.
   Parmi les paraphénylènediamines de formule (2) ci-dessus, on préfère tout particulièrement la paraphénylènediamine, la paratoluylènediamine, la 2-isopropylparaphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- **(II)** les bases doubles sont des composés comportant au moins deux noyaux aromatiques sur lesquels sont portés des groupements amino et/ou hydroxyle.
   Parmi les bases doubles utilisables à titre de bases d'oxydation dans les compositions tinctoriales conformes à l'invention, on peut notamment citer les composés répondant à la formule (3) suivante, et leurs sels d'addition avec un acide : 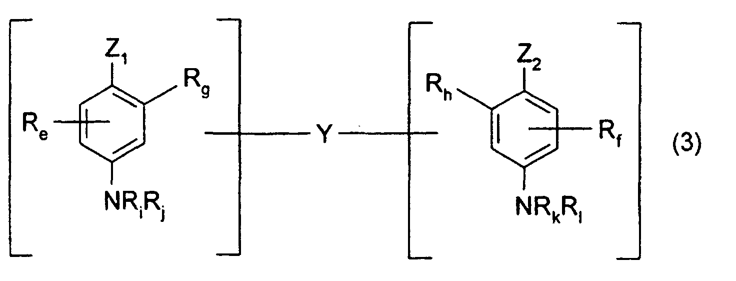 dans laquelle :
   - Z₁ et Z₂, identiques ou différents, représentent un radical hydroxyle ou -NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - Rₑ et R_{f} représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R_{g}, Rₕ, Rᵢ, Rⱼ, Rₖ et Rₗ, identiques ou différents, représentent un atome d'hydrogène, un bras de liaison Y ou un radical alkyle en C₁-C₄ ;
      étant entendu que les composés de formule (3) ne comportent qu'un seul bras de liaison Y par molécule.
      Parmi les groupements azotés de la formule (3) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
      Parmi les bases doubles de formules (3) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,6-dioxaoctane, et leurs sels d'addition avec un acide. Parmi ces bases doubles de formule (3), le N,N'-bis-(β-hydroxyéthyl) N,N'-bis-(4'-aminophényl) 1,3-diamino propanol, le 1,8-bis-(2,5-diaminophénoxy)-3,6-dioxaoctane ou l'un de leurs sels d'addition avec un acide sont particulièrement préférés.
   - **(III)** les para-aminophénols répondant à la formule (4) suivante, et leurs sels d'addition avec un acide : 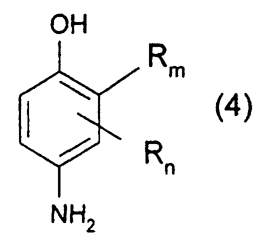 dans laquelle :
      Rₘ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
      Rₙ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en
      C₁-C₄, cyanoalkyle en C₁-C₄ ou alcoxy(C₁-C₄)alkyle(C₁-C₄).

      Parmi les para-aminophénols de formule (4) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
   - **(IV)** les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
   - **(V)** parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

   Parmi les dérivés pyridiniques, on peut plus particulièrement citer les composés décrits par exemple dans les brevets GB 1 026 978 et GB 1 153 196, comme la 2,5-diaminopyridine, la 2-(4-méthoxyphényl)amino-3-amino-pyridine, la 2,3-diamino-6-méthoxypyridine, la 2-(β-méthoxyéthyl)amino-3-amino-6-méthoxy pyridine, la 3,4-diamino-pyridine, et leurs sels d'addition avec un acide.
   Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolopyrimidiniques tels ceux mentionnés dans la demande dé brevet FR-A-2 750 048 et parmi lesquels on peut citer la pyrazolo-[1,5-a]-pyrimidine-3,7-diamine ; la 2,5-diméthylpyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol, le 2-(7-amino-pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthylpyrazolo-[1,5-a]-pyrimidine-3,7-diamine, la 2,6-diméthyl-pyrazolo-[1,5-a]-pyrimidine-3,7-diamine, la 2, 5, N7, N7-tetraméthyl-pyrazolo-[1,5-a]-pyrimidine-3,7-diamine, et leurs sels d'addition et leurs formes tautomères, lorsqu'il existe un équilibre tautomérique et leurs sels d'addition avec un acide.

Selon la présente invention, les bases d'oxydation additionnelles peuvent représenter de préférence de 0,0005 à 12% en poids environ du poids total de la composition tinctoriale

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ et encore plus préférentiellement entre 5 et 11 environ. Il peut être ajusté à la valeur désirée au moyen d'agents acidifiants ou alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (5) suivante : 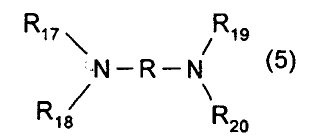 dans laquelle R est un reste propylène éventuellement substitué par un groupement hydroxyle ou un radical alkyle en C₁-C₄ ; R₁₇, R₁₈, R₁₉ et R₂₀, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques
ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à l'association conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant, cet agent oxydant pouvant être ajouté juste au moment de l'emploi à la composition tinctoriale ou au moyen d'une composition oxydante appliquée simultanément ou séquentiellement.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 60 minutes environ, de préférence 5 à 40 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates, percarbonates et persulfates, les peracides. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition tinctoriale qui est appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Selon une variante, on applique sur ces fibres dans un premier temps une composition contenant au moins le composé minéral, et dans un deuxième temps une composition contenant au moins un diamino-pyrazole, l'application de la composition contenant le ou les composés minéraux étant ou non suivie par une étape de rinçage, la couleur étant révélée à l'aide d'un agent oxydant.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Selon un mode de réalisation différent, le dispositif comprend au moins trois compartiments, un premier compartiment qui contient le composé minéral utile pour l'invention, un deuxième compartiment qui contient un diaminopyrazole, et un troisième compartiment qui contient une composition oxydante.

Les exemples qui suivent sont destinés à illustrer l'invention sans pour autant en limiter la portée.

### EXEMPLES

### EXEMPLES DE TEINTURE 1 à 4

On a préparé les compositions tinctoriales, conformes à l'invention, suivantes (teneurs en grammes, MA désigne Matière Active) :

| **EXEMPLES** | **1** | **2** | **3** | **4** |
|---|---|---|---|---|
| 4,5-diamino 1-βhydroxyéthyl pyrazole, 2 HCl (base d'oxydation) | 0,645 | 0,645 | 0,645 | 0,645 |
| 3-amino 6-méthyl phénol (coupleur) | 0,369 | 0,369 | 0.369 | 0,369 |
| Silice amorphe pyrogénée vendue sous la dénomination ACEMATT TS 100 par la Société DEGUSSA-HULS (composé minéral selon l'invention) | 0,3 | - | - | - |
| Alumine de faible granulométrie vendue sous la dénomination de ALUMINIUMOXIDC par la Société DEGUSSA-HULS(composé minéral selon l'invention) | - | 0,2 | - | - |
| Boehmite en suspension colloïdale à 75% vendue sous la dénomination DISPERSAL S par la Société CONDEA (composé minéral selon l'invention) | - | - | 0,15MA | - |
| Sulfate de zinc à 7 molécules d'eau (composé minéral selon l'invention) | - | - | - | 0,8 |
| Support de teinture commun | (**) | (**) | (**) | (**) |
| Eau déminéralisée q.s.p. | 100 g | 100 g | 100 g | 100 g |

| | | | | |
|---|---|---|---|---|
| (**) support de teinture commun contenant : - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3,0 g - Amine oléique à 2 moles d'oxyde d'éthylène vendue sous la dénomination commerciale ETHOMEEN 012 par la société AKZO 7,0 g - Laurylamino succinamate de diéthylaminopropyle, sel de sodium, à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - Antioxydant, séquestrant q.s. - Parfum, conservateur q.s. - Ammoniaque à 20 % de NH₃ 10 g | | | | |

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

On obtient dans tous les cas une nuance rouge puissante et tenace.avec une bonne innocuité.

### EXEMPLES DE TEINTURE 5 à 7

On a préparé les compositions tinctoriales, conformes à l'invention, suivantes (teneurs en grammes) :

| **EXEMPLE** | **5** | **6** | **7** |
|---|---|---|---|
| 4,5-diamino 1-éthyl 3-méthyl pyrazole, 2 HCl (base d'oxydation) | 0,639 | 0,639 | - |
| 3,4,5-triamino pyrazole, 2 HCl (base d'oxydation) | - | - | 0,667 |
| 3-amino 6-méthyl phénol (coupleur) | 0.369 | 0,369 | 0.369 |
| Boehmite en suspension colloidale à 75% vendue sous la dénomination DISPERSAL S par la Société CONDEA (composé minéral selon l'invention) | 0,15MA | - | 0,15MA |
| Sulfate de zinc à 7 molécules d'eau (composé minéral selon l'invention) | - | 0,2 | - |
| Support de teinture commun | (**) | (**) | (**) |
| Eau déminéralisée q.s.p. | 100 g | 100. g | 100 g |

| | | | |
|---|---|---|---|
| (**) support de teinture commun :identique à celui des exemples 1 à 4. | | | |

Au moment de l'emploi, on a mélangé chaque composition tinctoriale ci-dessus avec une quantité égale en poids d'une composition oxydante constituée par une solution d'eau oxygénée à 20 volumes (6 % en poids).

Chaque composition résultante a été appliquée pendant 30 minutes sur des mèches de cheveux gris naturels à 90 % de blancs. Les mèches de cheveux ont ensuite été rincées, lavées avec un shampooing standard puis séchées.

On obtient dans les trois cas une nuance puissante et tenace avec une bonne innocuité.

## Revendications

1. Composition pour la teinture d'oxydation des fibres kératiniques humaines et en particulier des fibres kératiniques humaines telles que les cheveux, comprenant, dans un milieu approprié pour la teinture :
- au moins une base d'oxydation choisie parmi les 4,5 ou 3,4-diamino pyrazoles et les triamino pyrazoles, et
- au moins un composé minéral sélectionné parmi les silices, les oxydes ou hydroxydes d'aluminium, les hydrosilicates d'aluminium, de magnésium et de fer, seul ou en mélange, et les sels de zinc ;
ladite composition ne contenant pas l'association d'une base d'oxydation du type 4,5-diaminopyrazole, d'un sel de zinc et d'un dérivé de 1,4-diazacycloheptane ou d'un de ses sels de formule suivante : 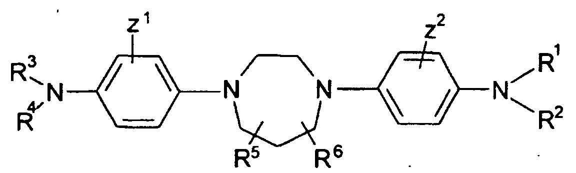 où R¹, R², R³ et R⁴ , identiques ou différents, désignent un hydrogène, un alkyle ou un hydroxyalkyle en C₁-C₄ ou bien un dihydroxyalkyle en C₂-C₄ ;
Z¹ et Z², identiques ou différents, désignent hydrogène, chlore, fluor, un alkyle en C₁-C₄, un hydroxyalkyle en C₁-C₄, un aminoalkyle en C₁-C₄, un alcoxy en C₁-C₄ ou un dihydroxyalkyle en C₂-C₄ ou un groupe allyle ;
R⁵ et R⁶ , identiques ou différents, désignent un hydrogène, un alkyle en C₁-C₄.

2. Composition selon la revendication 1, **caractérisée en ce que** la ou les silices sont choisies parmi les silices cristallines ou microcristallines et les silices non cristallines.

3. Composition selon la revendication 2, **caractérisée en ce que** la silice non cristalline est de la silice vitreuse.

4. Composition selon la revendication 2, **caractérisée en que** la silice non cristalline est choisie parmi les silices amorphes telles que les silices colloidales, les silicagels, les silices précipités et les fumées de silices.

5. Composition selon la revendication 1, **caractérisée en ce que** les hydroxydes d'aluminium sont choisis parmi les composés de composition Al(OH)₃ et les composés de composition AlOOH.

6. Composition selon la revendication 1, **caractérisée en ce que** les oxydes d'aluminium sont choisis parmi les alumines activées ou les alumines calcinées.

7. Composition selon la revendication 6, **caractérisée par** en ce que l'alumine activée est de l'alumine α.

8. Composition selon la revendication 1, **caractérisée en ce que** les hydrosilicates sont choisis parmi la kaolinite, la dicktite, la nacrite, l'halioysite-endellite la pyrophyllite ,la beidellite ,le talc ,le chrysotile ,la montmorillonite ,la saponite, la nontronite, la sauconite, l'amésite, la chamosite et l'illite.

9. Composition selon la revendication 1, **caractérisée en ce que** les sels de zinc sont choisis parmi les sels obtenus à partir d'un acide minéral.

10. Composition selon la revendication 9, **caractérisée en ce que** les sels de zinc sont choisis le chlorure, le sulfate, le nitrate, l'ortho phospate, l'ortho hydrogénophosphate, l'ortho dihydrogénophosphate, le carbonate, l'hydrogénocarbonate de zinc.

11. Composition selon la revendication 1, **caractérisée en ce que** les sels de zinc sont choisis parmi les sels obtenus à partir d'un acide organique.

12. Composition selon l'une quelconque des revendications 1 à 11, **caractérisée en ce que** le ou les composés minéraux représentent de 0,00001 à 10 % en poids environ du poids total de la composition tinctoriale, préférentiellement de 0,001 à 5 %, et plus préférentiellement de 0,001 à 3 % en poids environ de ce poids.

13. Composition selon l'une quelconque des revendications 1 à 12, **caractérisée en ce que** les 4,5 ou 3,4-diamino pyrazoles sont choisis parmi les 4,5 ou 3,4-diamino pyrazoles de formules (I) ou (II) suivantes et/ou leurs sels d'addition avec un acide : 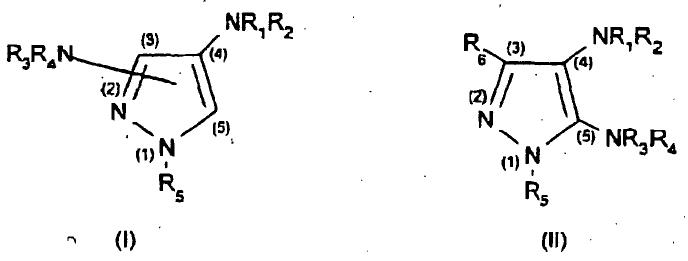 dans laquelle :
- R₁, R₂, R₃, R₄ et R₅, identiques ou différents, représentent un atome d'hydrogène : un radical alkyle en C₁-C₆ non substitué ou substitué par au moins un substituant choisi parmi OR, NHR, NRR', SR, SOR, SO₂R, COR, COOH, CONH₂, CONHR, CONRR', PO(OH)₂, SH, SO₃X, un hétérocycle non cationique, Cl, Br ou I, X désignant un atome d'hydrogène, Na, K, ou NH₄, et R et R', identique ou différents, représentant un alkyle ou alcényle en C₁-C₄ ; un radical hydroxyalkyle en C₂-C₄ ; un radical aminoalkyle en C₂-C₄ ; un radical phényle ; un radical phényle substitué par un atome d'halogène ou un radical alkyle en C₁-C₄, alcoxy en C₁-C₄, nitro, trifluorométhyle, amino ou alkylamino en C₁-C₄ ; un radical benzyle ; un radical benzyle substitué par un atome d'halogène ou par un radical alkyle en C₁-C₄, alcoxy en C₁-C₄, méthylènedioxy ou amino ; un radical 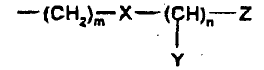
dans lequel m et n sont des nombres entiers, identiques ou différents, compris entre 0 et 3 inclusivement, X représente un atome d'oxygène ou bien le groupement NH, Y représente un atome d'hydrogène ou bien un radical alkyle en C₁-C₄, et Z représente un radical méthyle lorsque n est égal à 0, ou Z représente un radical alkyle en C₁-C₄, un groupement OR ou NR"R"' lorsque n est supérieur ou égal à 1, R" et R"', identiques ou différent, désignant un atome d'hydrogène, un radical alkyle en C₁-C₄ ; ou R₅ forme avec l'atome d'azote du groupement NR₃R₄ en position 5 un hétérocycle comprenant au moins 4 chaînons,
- R₆ représente un radical alkyle en C₁-C₆ ; un radical hydroxyalkyle en C₁-C₄ ; un radical aminoalkyle en C₁-C₄ ; un radical alkyle(C₁-C₄)aminoalkyle en C₁-C₄ ; un radical dialkyle (C₁-C₄)-aminoalkyle en C₁-C₄ ; un radical hydroxyalkyle (C₁-C₄)-aminoalkyle en C₁-C₄ ; un radical alcoxy(C₁-C₄)méthyle ; un radical phényle ; un radical phényle substitué par un atome d'halogène ou par un radical alkyle(C₁-C₄), alcoxy(C₁-C₄), nitro, trifluorométhyle, amino ou alkyl(C₁-C₄)amino ; un radical benzyle un radical benzyle substitué par un atome d'halogène ou par un radical alkyle(C₁-C₄), alcoxy(C₁-C₄), nitro, trifluorométhyle, amino ou alkyl(C₁-C₄)amino; un hétérocycle choisi parmi le thiophène, le furane et la pyridine, ou encore un radical -(CH₂)ₚ-O-(CH₂)_{q}-OR", dans lequel p et q sont des nombres entiers, identiques ou différents, compris entre 1 et 3 inclusivement et R" est tel que défini précédemment,
étant entendu que :
au moins un des radicaux R₁, R₂, R₃ et R₄ représente un atome d'hydrogène.

14. Composition selon l'une quelconque des revendications 1 à 12, **caractérisée en ce que** les triamino pyrazoles sont choisis parmi les composés de formule (III) suivante, et/ou leurs sels d'addition avec un acide : 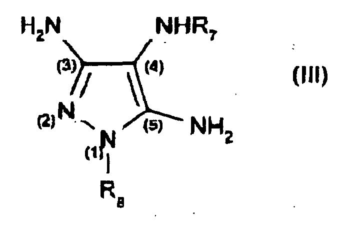 dans laquelle :
- R₇ et R₈, identiques ou différents, représentent un atome d'hydrogène, un radical alkyle en C₁-C₄ ou hydroxyalkyle en C₂-C₄.

15. Composition selon la revendication 13, **caractérisée en ce que** les 4,5 ou 3,4-diamino pyrazoles de formule (I) sont choisis parmi le 4,5-diamino 1-(4'-méthoxybenzyle) pyrazole, le 4,5-diamino 1-(4'-méthylbenzyle) pyrazole, la 4,5-diamino 1-(4'-chlorobenzyle) pyrazole, le 4,5-diamino 1-(3'-méthoxybenzyle) pyrazole, le 4-amino 1-(4'-méthoxybenzyle) 5-méthylamino pyrazole, le 4-amino 5-(β-hydroxyéthyl)amino 1-(4'-méthoxybenzyle) pyrazole, le 4-amino 5-(β-hydroxyéthyl)amino 1-méthyl pyrazole, le 4-amino (3) 5-méthylamino pyrazole, le 3-(5),4-diamino pyrazole, le 4,5-diamino 1-méthyl pyrazole, le 4,5-diamino 1-éthyl pyrazole, , le 4,5-diamino 1-isopropyl pyrazole le 4,5-diamino 1-hydroxyéthyl pyrazole, le 4,5-diamino 1-benzyl pyrazole, le 4-amino 5-hydroxyéthylamino 1-hydroxyéthyl pyrazole, le 4-diamino 5-méthylamino 1-hydroxyéthyl pyrazole, la 3-amino 4,5,7,8-tétrahydro pyrazolo [1,5-a] pyrimidine, le 7-amino 2,3-dihydro 1-H-imidazol [1,2-b] pyrazole, la 3-amino 8-méthyl 4,5,7,8-tétrahydropyrazolo [1,5-a] pyrimidine, l'acide 2-(4,5-diamino-pyrazol-1-yl)- 1-éthane sulfonique, le 2(4,5-diaminopyrazol-1-yl)-acétamide, l'acide 2-( 4,5-diamino-pyrazol-1-yl)-acétique, la 2-(2-diméthylamino-éthyl)-2H-pyrazole-3,4-diamine, la 2-(2-méthoxy-éthyl)-2H-pyrazole-3,4-diamine et leurs sels d'addition avec un acide.

16. Composition selon la revendication 15, **caractérisée par le fait que** les 4,5 ou 3,4-diamino pyrazoles de formule (I) sont choisis parmi
- le 4,5-diamino 1- benzyle pyrazole
- le 4,5-diamino 1-(4'-chlorobenzyle) pyrazole
- le 4,5-diamino 1-méthyl pyrazole
- le 4,5-diamino 1-hydroxyéthyl pyrazole
- la 2-(2-méthoxy-éthyl)-2H-pyrazole-3,4-diamine
et leurs sels d'addition avec un acide.

17. Composition selon la revendication 13, **caractérisée en ce que** les 4,5-diamino pyrazoles de formule (II) sont choisis parmi:
- le 1-benzyl 4,5-diamino 3-méthyl pyrazole,
- le 4,5-diamino 1-(β-hydroxyéthyl) 3-(4'-méthoxyphényl) pyrazole,
- le 4,5-diamino 1-(β-hydroxyéthyl) 3-(4'-méthylphényl) pyrazole,
- le 4,5-diamino 1-(β-hydroxyéthyl) 3-(3'-méthylphényl) pyrazole,
- le 4,5-diamino 3-méthyl 1-isopropyl pyrazole,
- le 4,5-diamino 3-(4'-méthoxyphényl) 1-isopropyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 3-hydroxyméthyl 1-méthyl pyrazole,
- le 4,5-diamino 1-éthyl 3-hydroxyméthyl pyrazole,
- le 4,5-diamino 3-hydroxyméthyl 1-isopropyl pyrazole,
- le 4,5-diamino 3-hydroxyméthyl 1-ter-butyl pyrazole,
- le 4,5-diamino 3-hydroxyméthyl 1-phényl pyrazole,
- le 4,5-diamino 3-hydroxyméthyl 1-(2'-méthoxyphényl) pyrazole,
- le 4,5-diamino 3-hydroxyméthyl 1-(3'-méthoxyphényl) pyrazole,
- le 4,5-diamino 3-hydroxyméthyl 1-(4'-méthoxyphényl) pyrazole,
- le 1-benzyl 4,5-diamino 3-hydroxyméthyl pyrazole,
- le 4,5-diamino 3-méthyl 1-(2'-méthoxyphényl) pyrazole,
- le 4,5-diamino 3-méthyl 1-(3'-méthoxyphényl) pyrazole,
- le 4,5-diamino 3-méthyl 1-(4'-méthoxyphényl) pyrazole,
- le 3-aminométhyl 4,5-diamino 1-méthyl pyrazole,
- le 3-aminométhyl 4,5-diamino 1-éthyl pyrazole,
- le 3-aminométhyl 4,5-diamino 1-isopropyl pyrazole,
- le 3-aminométhyl 4,5-diamino 1-ter-butyl pyrazole,
- le 4,5-diamino 3-diméthylaminométhyl 1-méthyl pyrazole,
- le 4,5-diamino 3-diméthylaminométhyl 1-éthyl pyrazole,
- le 4,5-diamino-3-diméthylaminométhyl 1-isopropyl pyrazole,
- le 4,5-diamino 3-diméthylaminométhyl 1-ter-butyl pyrazole,
- le 4,5-diamino 3-éthylaminométhyl 1-méthyl pyrazole,
- le 4,5-diamino 3-éthylaminométhyl 1-éthyl pyrazole,
- le 4,5-diamino 3-éthylaminométhyl 1-isopropyl pyrazole,
- le 4,5-diamino 3-éthylaminométhyl 1-ter-butyl pyrazole,
- le 4,5-diamino 3-méthylaminométhyl 1-méthyl pyrazole,
- le 4,5-diamino 3-méthylaminométhyl 1-isopropyl pyrazole,
- le 4,5-diamino 1-éthyl 3-méthylaminométhyl pyrazole,
- le 1-ter-butyl 4,5-diamino 3-méthylaminométhyl pyrazole,
- le 4,5-diamino 3-[(β-hydroxyéthyl)aminométhyl] 1-méthyl pyrazole,
- le 4,5-diamino 3-[(β-hydroxyéthyl)aminométhyl] 1-isopropyl pyrazole,
- le 4,5-diamino 1-éthyl 3-[(β-hydroxyéthyl)aminométhyl] pyrazole,
- le 1-ter-butyl 4,5-diamino 3-[(β-hydroxyéthyl)aminométhyl] pyrazole,
- le 4-amino 5-(β-hydroxyéthyl)amino 1,3-diméthyl pyrazole,
- le 4-amino 5-(β-hydroxyéthyl)amino 1-isopropyl 3-méthyl pyrazole,
- le 4-amino 5-(β-hydroxyéthyl)amino 1-éthyl 3-méthyl pyrazole,
- le 4-amino 5-(β-hydroxyéthyl)amino 1-ter-butyl 3-méthyl pyrazole,
- le 4-amino 5-(β-hydroxyéthyl)amino 1-phényl 3-méthyl pyrazole,
- le 4-amino 5-(β-hydroxyéthyl)amino 1-(2-méthoxyphényl) 3-méthyl pyrazole,
- le 4-amino 5-(β-hydroxyéthyl)amino 1-(3-méthoxyphényl) 3-méthyl pyrazole,
- le 4-amino 5-(β-hydroxyéthyl)amino 1-(4-méthoxyphényl) 3-méthyl pyrazole,
- le 4-amino 5-(β-hydroxyéthyl)amino 1-benzyl 3-méthyl pyrazole,
- le 4-amino 1-éthyl 3-méthyl 5-méthylamino pyrazole,
- le 4-amino 1-ter-butyl 3-méthyl 5-méthylamino pyrazole,
- le 4,5-diamino 1,3-diméthyl pyrazole,
- le 4,5-diamino 3-tert.butyl 1-méthyl pyrazole,
- le 4,5-diamino 1-tert.butyl 3-méthyl pyrazole,
- le 4,5-diamino 1-méthyl 3-phényl pyrazole,
- le 4,5-diamino 1-(β-hydroxyéthyl) 3-méthyl pyrazole,
- le 4,5-diamino 1-(β-hydroxyéthyl) 3-phényl pyrazole,
- le 4,5-diamino 1-méthyl 3-(2'-chlorophényl) pyrazole,
- le 4,5-diamino 1-méthyl 3-(4'-chlorophényl) pyrazole,
- le 4,5-diamino 1-méthyl 3-(3'-trifluorométhylphényl) pyrazole,
- le 4,5-diamino 1,3-diphényl pyrazole,
- le 4,5-diamino 3-méthyl 1-phényl pyrazole,
- le 4-amino 1,3-diméthyl 5-phénylamino pyrazole,
- le 4-amino 1-éthyl 3-méthyl 5-phénylamino pyrazole,
- le 4-amino 1,3-diméthyl 5-méthylamino pyrazole,
- le 4-amino 3-méthyl 1-isopropyl 5-méthylamino pyrazole,
- le 4-amino 3-isobutoxyméthyl 1-méthyl 5-méthylamino pyrazole,
- le 4-amino 3-méthoxyéthoxyméthyl 1-méthyl 5-méthylamino pyrazole,
- le 4-amino 3-hydroxyméthyl 1-méthyl 5-méthylamino pyrazole,
- le 4-amino 1,3-diphényl 5-phénylamino pyrazole,
- le 4-amino 3-méthyl 5-méthylamino 1-phényl pyrazole,
- le 4-amino 1,3-diméthyl 5-hydrazino pyrazole,
- le 5-amino 3-méthyl 4-méthylamino 1-phényl pyrazole,
- le 5-amino 1-méthyl 4-(N,N-méthylphényl)amino 3-(4'-chlorophényl) pyrazole,
- le 5-amino 3-éthyl 1-méthyl 4-(N,N-méthylphényl)amino pyrazole,
- le 5-amino 1-méthyl 4-(N,N-méthylphényl)amino 3-phényl pyrazole,
- le 5-amino 3-éthyl 4-(N,N-méthylphényl)amino pyrazole,
- le 5-amino 4-(N,N-méthylphényl)amino 3-phényl pyrazole,
- le 5-amino 4-(N,N-méthylphényl)amino 3-(4'-méthylphényl) pyrazole,
- le 5-amino 3-(4'-chlorophényl) 4-(N,N-méthylphényl)amino pyrazole,
- le 5-amino 3-(4'-méthoxyphényl) 4-(N,N-méthylphényl)amino pyrazole,
- le 4-amino 5-méthylamino 3-phényl pyrazole,
- le 4-amino 5-éthylamino 3-phényl pyrazole,
- le 4-amino 5-éthylamino 3-(4'-méthylphényl) pyrazole,
- le 4-amino 3-phényl 5-propylamino pyrazole,
- le 4-amino 5-butylamino 3-phényl pyrazole,
- le 4-amino 3-phényl 5-phénylamino pyrazole,
- le 4-amino 5-benzylamino 3-phényl pyrazole,
- le 4-amino 5-(4'-chlorophényl)amino 3-phényl pyrazole,
- le 4-amino 3-(4'-chlorophényl) 5-phénylamino pyrazole,
- le 4-amino 3-(4'-méthoxyphényl) 5-phénylamino pyrazole,
- le 1-(4'-chlorobenzyl) 4,5-diamino 3-méthyl pyrazole,
- le 4,5-diamino 3-hydroxyméthyl 1-isopropyl pyrazole,
- le 4-amino 1-éthyl 3-méthyl 5-méthylamino pyrazole,
- le 4-amino 5-(2'-aminoéthyl)amino 1,3-diméthyl pyrazole,
et leurs sels d'addition avec un acide.

18. Composition selon la revendication 13, **caractérisée en ce que** les 4,5-diamino pyrazoles de formule (II) sont choisis parmi :
- le 4,5-diamino 1,3-diméthyl pyrazole,
- le 4,5-diamino 3-méthyl 1-phényl pyrazole,
- le 4,5-diamino 1-méthyl 3-phényl pyrazole,
- le 4-amino 1,3-diméthyl 5-hydrazino pyrazole,
- le 1-benzyl 4,5-diamino 3-méthyl pyrazole,
- le 4,5-diamino 3-tert-butyl 1-méthyl pyrazole,
- le 4,5-diamino 1-tert-butyl 3-méthyl pyrazole,
- le 4,5-diamino 1-(β-hydroxyéthyl) 3-méthyl pyrazole,
- le 4,5-diamino 1-éthyl 3-méthyl pyrazole,
- le 4,5-diamino 1-éthyl 3-(4'-méthoxyphényl) pyrazole,
- le 4,5-diamino 1-éthyl 3-hydroxyméthyl pyrazole,
- le 4,5-diamino 3-hydroxyméthyl 1-méthyl pyrazole,
- le 4,5-diamino 3-hydroxyméthyl 1-isopropyl pyrazole,
- le 4,5-diamino 3-méthyl 1-isopropyl pyrazole,
- le 4-amino 5-(2'-aminoéthyl)amino 1,3-diméthyl pyrazole,
et leurs sels d'addition avec un acide.

19. Composition selon la revendication 14, **caractérisée en ce que** les triamino pyrazoles de formule (III) sont choisis parmi le 3,4,5-triamino pyrazole, le 1-méthyl 3,4,5-triamino pyrazole, le 3,5-diamino, 1-méthyl 4-méthylamino pyrazole, et le 3,5-diamino 4-(β-hydroxyéthyl)amino 1-méthyl pyrazole, et leurs sels d'addition avec un acide.

20. Composition selon l'une quelconque des revendications précédentes 1 à 19, **caractérisée en ce que** le ou les 4,5 ou 3,4-diamino pyrazoles et/ou le ou les triamino pyrazoles et/ou le ou les sels d'addition avec un acide correspondants représentent de 0,0005 à 12 % en poids du poids total de la composition tinctoriale.

21. Composition selon la revendication 20, **caractérisée en ce que** le ou les 4,5 ou 3,4-diamino pyrazoles et/ou le ou les triamino pyrazoles et/ou le ou les sels d'addition avec un acide correspondants représentent de 0,005 à 6 % en poids du poids total de la composition tinctoriale.

22. Composition selon l'une quelconque des revendications précédentes 1 à 21, **caractérisée en ce que** le rapport pondéral du ou des composés minéraux avec le ou les 4,5 ou 3,4-diamino pyrazoles et/ou le ou les triamino pyrazoles et/ou le ou les sels d'addition avec un acide est compris entre 0,001 et 100 et de préférence entre 0,01 et 10.

23. Composition selon l'une quelconque des revendications précédentes 1 à 22, **caractérisée en ce que** les sels d'addition avec un acide sont choisis parmi les chlorhydrates, les bromhydrates, les sulfates et les tartrates, les lactates et les acétates.

24. Composition selon l'une quelconque des revendications précédentes 1 à 23, **caractérisée en ce qu'**elle contient au moins un coupleur.

25. Composition selon la revendication 24, **caractérisée en ce que** le ou les coupleurs représentent de 0,0001 à 10% en poids environ du poids total de la composition tinctoriale et encore plus préférentiellement de 0,005 à 5 % en poids environ de ce poids.

26. Composition selon l'une quelconque des revendications précédentes 1 à 25, **caractérisée par le fait qu'**elle contient au moins une base d'oxydation additionnelle autre que les pyrazoles définis dans les revendications 1 à 17.

27. Composition selon la revendication 26 , **caractérisée en ce que** la ou les bases d'oxydation additionnelles représentent de 0,0005 à 12% en poids environ du poids total de la composition tinctoriale

28. Composition selon l'une quelconque des revendications précédentes 1 à 27, **caractérisée par le fait qu'**elle présente un pH compris entre 3 et 12.

29. Composition selon l'une quelconque des revendications précédentes 1 à 28, **caractérisée en ce qu'**elle se présente sous forme de liquides, de crèmes, de gels, ou sous toute autre forme appropriée pour réaliser une teinture des fibres kératiniques, et notamment des cheveux humains.

30. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 1 à 29, et que l'on révèle la couleur à pH acide, neutre ou alcalin à l'aide d'un agent oxydant.

31. Procédé selon la revendication 30, **caractérisé en ce que** l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides.

32. Procédé de teinture des fibres kératiniques et en particulier des fibres kératiniques humaines telles que les cheveux **caractérisé par le fait que** l'on applique sur ces fibres dans un premier temps une composition contenant au moins un composé minéral tel que défini dans l'une quelconque des revendications 1 à 29, dans un deuxième temps une composition contenant au moins un diamino-pyrazole tel que défini dans l'une quelconque des revendications 1 à 29, l'application de la composition contenant le ou les composés minéraux étant ou non suivie par une étape de rinçage, la couleur étant révélée à l'aide d'un agent oxydant.

33. Dispositif à plusieurs compartiments comprenant un premier compartiment renfermant une composition tinctoriale telle que définie à l'une quelconque des revendications 1 à 29 et un second compartiment renfermant une composition oxydante.

34. Dispositif à plusieurs compartiments comprenant un premier compartiment renfermant un composé minéral tel que défini dans l'une quelconque des revendications 1 à 29, un deuxième compartiment renfermant un diaminopyrazole tel que défini dans l'une quelconque des revendications 1 à 29, et un troisième compartiment renfermant une composition oxydante.

## Patentansprüche

1. Zusammensetzung zum oxidativen Färben von menschlichen Keratinfasern und insbesondere menschlichen Keratinfasern, wie Haaren, die in einem zum Färben geeigneten Träger enthält:
- mindestens eine Oxidationsbase, die unter den 4,5- oder 3,4-Diaminopyrazolen und den Triaminopyrazolen ausgewählt ist, und
- mindestens eine anorganische Verbindung, die unter den Siliciumdioxid-Verbindungen, Aluminiumoxiden oder Aluminiumhydroxiden, Hydrosilicaten von Aluminium, Magnesium und Eisen einzeln oder im Gemisch und Zinksalzen ausgewählt ist;
wobei die Zusammensetzung die folgende Kombination nicht enthält: eine Oxidationsbase vom Typ 4,5-Diaminopyrazol, ein Zinksalz und ein 1,4-Diazocycloheptanderivat oder eines seiner Salze der folgenden Formel: 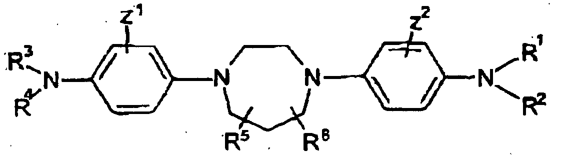 worin die Gruppen R¹, R², R³ und R⁴, die gleich oder verschieden sind, Wasserstoff, Alkyl oder Hydroxyalkyl mit 1 bis 4 Kohlenstoffatomen oder Dihydroxyalkyl mit 2 bis 4 Kohlenstoffatomen bedeuten;
Z¹ und Z², die gleich oder verschieden sind, Wasserstoff, Chlor, Fluor, C₁₋₄-Alkyl, C₁₋₄-Hydroxyalkyl, C₁₋₄-Aminoalkyl, C₁₋₄-Alkoxy oder C₂₋₄-Dihydroxyalkyl oder Allyl bedeuten; und
R⁵ und R⁶, die gleich oder verschieden sind, Wasserstoff oder C₁₋₄-Alkyl bedeuten.

2. Zusammensetzung nach Anspruch 1, **dadurch gekennzeichnet, dass** die Siliciumdioxid-Verbindungen unter den kristallinen oder mikrokristallinen Siliciumdioxiden oder nicht kristallinen Siliciumdioxiden ausgewählt sind.

3. Zusammensetzung nach Anspruch 2, **dadurch gekennzeichnet, dass** es sich bei dem nicht kristallinen Siliciumdioxid um Quarzglas handelt.

4. Zusammensetzung nach Anspruch 2, **dadurch gekennzeichnet, dass** das nicht kristalline Siliciumdioxid unter den amorphen Siliciumdioxiden, wie kolloidalen Kieselsäuren, Silicagelen, Fällungskieselsäuren und flammenhydrolytisch hergestellten Kieselsäuren ausgewählt ist.

5. Zusammensetzung nach Anspruch 1, **dadurch gekennzeichnet, dass** die Aluminiumhydroxide unter den Verbindungen der Zusammensetzung Al(OH)₃ und den Verbindungen der Zusammensetzung AlOOH ausgewählt sind.

6. Zusammensetzung nach Anspruch 1, **dadurch gekennzeichnet, dass** die Aluminiumoxide unter den aktiven Aluminiumoxiden oder calcinierten Aluminiumoxiden ausgewählt sind.

7. Zusammensetzung nach Anspruch 6, **dadurch gekennzeichnet, dass** das aktive Aluminiumoxid das α-Aluminiumoxid ist.

8. Zusammensetzung nach Anspruch 1, **dadurch gekennzeichnet, dass** die Hydrosilicate unter Kaolinit, Dickit, Nacrit, Halloysit-Endellit, Pyrophillit, Beidellit, Talk, Chrysotil, Montmorillonit, Saponit, Nontronit, Sauconit, Amesit, Chamosit und Illit ausgewählt sind.

9. Zusammensetzung nach Anspruch 1, **dadurch gekennzeichnet, dass** die Zinksalze unter den Salzen ausgewählt sind, die mit einer anorganischen Säure erhalten werden.

10. Zusammensetzung nach Anspruch 9, **dadurch gekennzeichnet, dass** die Zinksalze unter dem Chlorid, Sulfat, Nitrat, Orthophosphat, Orthohydrogenphosphat, Orthodihydrogenphosphat, Carbonat und Hydrogencarbonat von Zink ausgewählt sind.

11. Zusammensetzung nach Anspruch 1, **dadurch gekennzeichnet, dass** die Zinksalze unter den Salzen ausgewählt sind, die mit einer organischen Säure erhalten werden.

12. Zusammensetzung nach einem der Ansprüche 1 bis 11, **dadurch gekennzeichnet, dass** die anorganische(n) Verbindung(en) etwa 0,00001 bis 10 Gew.-% des Gesamtgewichts der Farbmittelzusammensetzung, vorzugsweise 0,001 bis 5 % und noch bevorzugter etwa 0,001 bis 3 Gew.-% des Gesamtgewichts ausmachen.

13. Zusammensetzung nach einem der Ansprüche 1 bis 12, **dadurch gekennzeichnet, dass** die 4,5- oder 3,4-Diaminopyrazole unter den 4,5- oder 3,4-Diaminopyrazolen der folgenden Formeln (I) oder (II) und/oder deren Additionssalzen mit einer Säure ausgewählt sind: 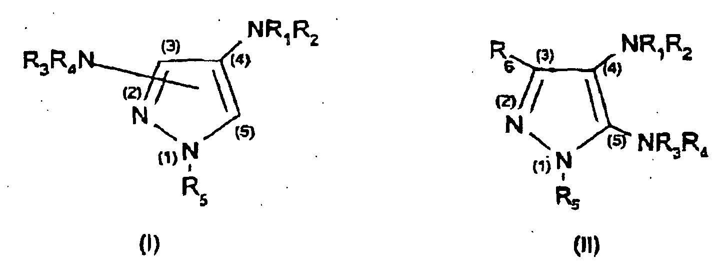 worin bedeuten:
- die Gruppen R₁, R₂, R₃, R₄ und R₅, die gleich oder verschieden sind, ein Wasserstoffatom; eine C₁₋₆-Alkylgruppe, die unsubstituiert vorliegt oder mit mindestens einem Substituenten substituiert ist, der unter OR, NHR, NRR', SR, SOR, SO₂R, COR, COOH, CONH₂, CONHR, CONRR', PO(OH)₂, SH, SO₃X, einem nicht kationischen Heterocyclus, Cl, Br oder I ausgewählt ist, wobei X ein Wasserstoffatom, Na, K oder NH₄ bedeutet und die Gruppen R und R', die gleich oder verschieden sind, eine Alkyl- oder Alkenylgruppe mit 1 bis 4 Kohlenstoffatomen bedeuten; C₂₋₄-Hydroxyalkyl; C₂₋₄-Aminoalkyl; Phenyl; eine Phenylgruppe, die mit einem Halogenatom oder C₁₋₄-Alkyl, C₁₋₄-Alkoxy, Nitro, Trifluormethyl, Amino oder C₁₋₄-Alkylamino substituiert ist; Benzyl; eine Benzylgruppe, die mit einem Halogenatom oder C₁₋₄-Alkyl, C₁₋₄-Alkoxy, Methylendioxy oder Amino substituiert ist; eine Gruppe 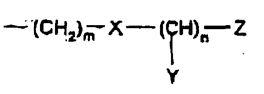
worin m und n, die gleich oder verschieden sind, 0 oder ganze Zahlen von 1 bis 3 bedeuten, X ein Sauerstoffatom oder die Gruppe NH bedeutet, Y ein Wasserstoffatom oder eine C₁₋₄-Alkylgruppe ist und Z eine Methylgruppe bedeutet, wenn n 0 ist, oder Z eine C₁₋₄-Alkylgruppe, OR oder NR"R'" bedeutet, wenn n 1 bedeutet oder darüber liegt, wobei R" und R'", die gleich oder verschieden sind, ein Wasserstoffatom oder C₁₋₄-Alkyl bedeuten; oder R₅ bildet mit dem Stickstoffatom der Gruppe NR₃R₄ in 5-Stellung einen Heterocyclus, der mindestens 4 Ringatome aufweist;
- R₆ C₁₋₆-Alkyl; C₁₋₄-Hydroxyalkyl; C₁₋₄-Aminoalkyl; Alkyl(C₁₋₄)-aminoalkyl(C₁₋₄); Dialkyl(C₁₋₄)aminoalkyl(C₁₋₄); Hydroxyalkyl(C₁₋₄)aminoalkyl(C₁₋₄); Alkoxy(C₁₋₄)methyl; Phenyl; eine Phenylgruppe, die mit einem Halogenatom oder Alkyl(C₁₋₄), Alkoxy(C₁₋₄), Nitro, Trifluormethyl, Amino oder Alkyl(C₁₋₄)amino substituiert ist; Benzyl; eine Benzylgruppe, die mit einem Halogenatom oder Alkyl(C₁₋₄), Alkoxy(C₁₋₄), Nitro, Trifluormethyl, Amino oder Alkyl(C₁₋₄)amino substituiert ist; einen Heterocyclus, der unter Thiophen, Furan und Pyridin ausgewählt ist, oder eine Gruppe -(CH₂)ₚ-O-(CH₂)_{q}-OR", wobei p und q, die gleich oder voneinander verschieden sind, ganze Zahlen im Bereich von 1 bis 3 bedeuten und R" die oben angegebenen Bedeutungen aufweist,
mit der Maßgabe, dass:
- mindestens eine der Gruppen R₁, R₂, R₃ und R₄ ein Wasserstoffatom bedeutet.

14. Zusammensetzung nach einem der Ansprüche 1 bis 12, **dadurch gekennzeichnet, dass** die Triaminopyrazole unter den Verbindungen der folgenden Formel (III) und/oder den Additionssalzen mit einer Säure ausgewählt sind: 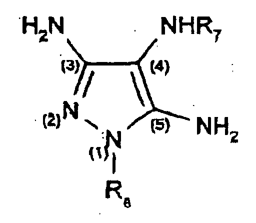 worin bedeuten:
- R₇ und R₈, die gleich oder verschieden sind, ein Wasserstoff atom, C₁₋₄-Alkyl oder C₂₋₄-Hydroxyalkyl.

15. Zusammensetzung nach Anspruch 13, **dadurch gekennzeichnet, dass** die 4,5- oder 3,4-Diaminopyrazole der Formel (I) ausgewählt sind unter: 4,5-Diamino-1-(4'-methoxybenzyl)-pyrazol, 4,5-Diamino-1-(4'-methylbenzyl)-pyrazol, 4,5-Diamino-1-(4'-chlorbenzyl)-pyrazol, 4,5-Diamino-1-(3'-methoxybenzyl)-pyrazol, 4-Amino-1-(4'-methoxybenzyl)-5-methylamino-pyrazol, 4-Amino-5-(β-hydroxyethyl)-amino-1-(4'-methoxybenzyl)-pyrazol, 4-Amino-5-(β-hydroxyethyl)-amino-1-methyl-pyrazol, 4-Amino-(3)5-methylamino-pyrazol, 3(S),4-Diamino-pyrazol, 4,5-Diamino-1-methyl-pyrazol, 4,5-Diamino-1-ethyl-pyrazol, 4,5-Diamino-1-isopropyl-pyrazol, 4,5-Diamino-1-hydroxyethyl-pyrazol, 4,5-Diamino-1-benzyl-pyrazol, 4-Diamino-5-hydroxyethylamino-1-hydroxyethyl-pyrazol, 4- amino-5-methylamino-1-hydroxyethylpyrazol, 3-Amino-4,5,7,8-tetrahydro-pyrazolo[1,5-a]pyrimidin, 7-Amino-2,3-dihydro-1H-imidazol[1,2-b]pyrazol, 3-Amino-8-methyl-4,5,7-8-tetrahydropyrazolo[1,5-a]pyrimidin, 2-(4,5-Diamino-pyrazol-1-yl)-1-ethan-sulfonsäure, 2-(4,5-Diaminopyrazol-1-yl)-acetamid, 2-(4,5-Diamino-pyrazol-1-yl)-essigsäure, 2-(2-Dimethylamino-ethyl)-2H-pyrazol-3,4-diamin, 2-(2-Methoxyethyl)-2H-pyrazol-3,4-diamin und deren Additionssalze mit einer Säure.

16. Zusammensetzung nach Anspruch 15, **dadurch gekennzeichnet, dass** die 4,5- oder 3,4-Diaminopyrazole der Formel (I) ausgewählt sind unter:
- 4,5-Diamino-1-benzyl-pyrazol,
- 4,5-Diamino-1-(4'-chlorbenzyl)-pyrazol,
- 4,5-Diamino-1-methyl-pyrazol,
- 4,5-Diamino-1-hydroxyethyl-pyrazol,
- 2-(2-Methoxy-ethyl)-2H-pyrazol-3,4-diamin
und deren Additionssalzen mit einer Säure.

17. Zusammensetzung nach Anspruch 13, **dadurch gekennzeichnet, dass** die 4,5-Diaminopyrazole der Formel (II) ausgewählt sind unter:
- 1-Benzyl-4,5-diamino-3-methyl-pyrazol,
- 4,5-Diamino-1-(β-hydroxyethyl)-3-(4'-methoxyphenyl)-pyrazol,
- 4,5-Diamino-1-(β-hydroxyethyl)-3-(4'-methylphenyl)-pyrazol,
- 4,5-Diamino-1-(β-hydroxyethyl)-3-(3'-methylphenyl)-pyrazol,
- 4,5-Diamino-3-methyl-1-isopropyl-pyrazol,
- 4,5-Diamino-3-(4'-methoxyphenyl)-1-isopropyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-3-hydroxymethyl-1-methyl-pyrazol,
- 4,5-Diamino-1-ethyl-3-hydroxymethyl-pyrazol,
- 4,5-Diamino-3-hydroxymethyl-1-isopropyl-pyrazol,
- 4,5-Diamino-3-hydroxymethyl-1-*t*-butyl-pyrazol,
- 4,5-Diamino-3-hydroxymethyl-1-phenyl-pyrazol,
- 4,5-Diamino-3-hydroxymethyl-1-(2'-methoxyphenyl)-pyrazol,
- 4,5-Diamino-3-hydroxymethyl-1-(3'-methoxyphenyl)-pyrazol,
- 4,5-Diamino-3-hydroxymethyl-1-(4'-methoxyphenyl)-pyrazol,
- 1-Benzyl-4,5-diamino-3-hydroxymethyl-pyrazol,
- 4,5-Diamino-3-methyl-1-(2'-methoxyphenyl)-pyrazol,
- 4,5-Diamino-3-methyl-1-(3'-methoxyphenyl)-pyrazol,
- 4,5-Diamino-3-methyl-1-(4'-methoxyphenyl)-pyrazol,
- 3-Aminomethyl-4,5-diamino-1-methyl-pyrazol,
- 3-Aminomethyl-4,5-diamino-1-ethyl-pyrazol,
- 3-Aminomethyl-4,5-diamino-1-isopropyl-pyrazol,
- 3-Aminomethyl-4,5-diamino-1-*t*-butyl-pyrazol,
- 4,5-Diamino-3-dimethylaminomethyl-1-methyl-pyrazol,
- 4,5-Diamino-3-dimethylaminomethyl-1-ethyl-pyrazol,
- 4,5-Diamino-3-dimethylaminomethyl-1-isopropyl-pyrazol,
- 4,5-Diamino-3-dimethylaminomethyl-1-*t*-butyl-pyrazol,
- 4,5-Diamino-3-ethylaminomethyl-1-methyl-pyrazol,
- 4,5-Diamino-3-ethylaminomethyl-1-ethyl-pyrazol,
- 4,5-Diamino-3-ethylaminomethyl-1-isopropyl-pyrazol,
- 4,5-Diamino-3-ethylaminomethyl-1-*t*-butyl-pyrazol,
- 4,5-Diamino-3-methylaminomethyl-1-methyl-pyrazol,
- 4,5-Diamino-3-methylaminomethyl-1-isopropyl-pyrazol,
- 4,5-Diamino-1-ethyl-3-methylaminomethyl-pyrazol,
- 1-*t*-Butyl-4,5-diamino-3-methylaminomethyl-pyrazol,
- 4,5-Diamino-3-[(β-hydroxyethyl)aminomethyl]-1-methylpyrazol,
- 4,5-Diamino-3-[(β-hydroxyethyl)aminomethyl]-1-isopropylpyrazol,
- 4,5-Diamino-1-ethyl-3-[(β-hydroxyethyl)aminomethyl]-pyrazol,
- 1-*t*-Butyl-4,5-diamino-3-[(β-hydroxyethyl)aminomethyl]-pyrazol,
- 4-Amino-5-(β-hydroxyethyl)amino-1,3-dimethyl-pyrazol,
- 4-Amino-5-(β-hydroxyethyl)amino-1-isopropyl-3-methylpyrazol,
- 4-Amino-5-(β-hydroxyethyl)amino-1-ethyl-3-methyl-pyrazol,
- 4-Amino-5-(β-hydroxyethyl)amino-1-*t*-butyl-3-methyl-pyrazol,
- 4-Amino-5-(β-hydroxyethyl)amino-1-phenyl-3-methyl-pyrazol,
- 4-Amino-5-(β-hydroxyethyl)amino-1-(2-methoxyphenyl)-3-methyl-pyrazol,
- 4-Amino-5-(β-hydroxyethyl)amino-1-(3-methoxyphenyl)-3-methyl-pyrazol,
- 4-Amino-5-(β-hydroxyethyl)amino-1-(4-methoxyphenyl)-3-methyl-pyrazol,
- 4-Amino-5-(β-hydroxyethyl)amino-1-benzyl-3-methyl-pyrazol,
- 4-Amino-1-ethyl-3-methyl-5-methylamino-pyrazol,
- 4-Amino-1-*t*-butyl-3-methyl-5-methylamino-pyrazol,
- 4,5-Diamino-1,3-dimethyl-pyrazol,
- 4,5-Diamino-3-*t*-butyl-1-methyl-pyrazol,
- 4,5-Diamino-1-*t*-butyl-3-methyl-pyrazol,
- 4,5-Diamino-1-methyl-3-phenyl-pyrazol,
- 4,5-Diamino-1-(β-hydroxyethyl)-3-methyl-pyrazol,
- 4,5-Diamino-1-(β-hydroxyethyl)-3-phenyl-pyrazol,
- 4,5-Diamino-1-methyl-3-(2'-chlorphenyl)-pyrazol,
- 4,5-Diamino-1-methyl-3-(4'-chlorphenyl)-pyrazol,
- 4,5-Diamino-1-methyl-3-(3'-trifluormethylphenyl)-pyrazol,
- 4,5-Diamino-1,3-diphenyl-pyrazol,
- 4,5-Diamino-3-methyl-1-phenyl-pyrazol,
- 4-Amino-1,3-dimethyl-5-phenylamino-pyrazol,
- 4-Amino-1-ethyl-3-methyl-5-phenylamino-pyrazol,
- 4-Amino-1,3-dimethyl-5-methylamino-pyrazol,
- 4-Amino-3-methyl-1-isopropyl-5-methylamino-pyrazol,
- 4-Amino-3-isobutoxymethyl-1-methyl-5-methylamino-pyrazol,
- 4-Amino-3-methoxyethoxymethyl-1-methyl-5-methylaminopyrazol,
- 4-Amino-3-hydroxymethyl-1-methyl-5-methylamino-pyrazol,
- 4-Amino-1,3-diphenyl-5-phenylamino-pyrazol,
- 4-Amino-3-methyl-5-methylamino-1-phenyl-pyrazol,
- 4-Amino-1,3-dimethyl-5-hydrazino-pyrazol,
- 5-Amino-3-methyl-4-methylamino-1-phenyl-pyrazol,
- 5-Amino-1-methyl-4-(N,N-methylphenyl)amino-3-(4'-chlorphenyl)-pyrazol,
- 5-Amino-3-ethyl-1-methyl-4-(N,N-methylphenyl)amino-pyrazol,
- 5-Amino-1-methyl-4-(N,N-methylphenyl)amino-3-phenylpyrazol,
- 5-Amino-3-ethyl-4-(N,N-methylphenyl)amino-pyrazol,
- 5-Amino-4-(N,N-methylphenyl)amino-3-phenyl-pyrazol,
- 5-Amino-4-(N,N-methylphenyl)amino-3-(4'-methylphenyl)-pyrazol,
- 5-Amino-3-(4'-chlorphenyl)-4-(N,N-methylphenyl)aminopyrazol,
- 5-Amino-3-(4'-methoxyphenyl)-4-(N,N-methylphenyl)aminopyrazol,
- 4-Amino-5-methylamino-3-phenyl-pyrazol,
- 4-Amino-5-ethylamino-3-phenyl-pyrazol,
- 4-Amino-5-ethylamino-3-(4'-methylphenyl)-pyrazol,
- 4-Amino-3-phenyl-5-propylamino-pyrazol,
- 4-Amino-5-butylamino-3-phenyl-pyrazol,
- 4-Amino-3-phenyl-5-phenylamino-pyrazol,
- 4-Amino-5-benzylamino-3-phenyl-pyrazol,
- 4-Amino-5-(4'-chlorphenyl)-amino-3-phenyl-pyrazol,
- 4-Amino-3-(4'-chlorphenyl)-5-phenylamino-pyrazol,
- 4-Amino-5-(4'-methoxyphenyl)-5-phenylamino-pyrazol,
- 1-(4'-Chlorbenzyl)-4,5-diamino-3-methyl-pyrazol,
- 4,5-Diamino-3-hydroxymethyl-1-isopropyl-pyrazol,
- 4-Amino-1-ethyl-3-methyl-5-methylamino-pyrazol,
- 4-Amino-5-(2'-aminoethyl)amino-1,3-dimethyl-pyrazol
und deren Additionssalzen mit einer Säure.

18. Zusammensetzung nach Anspruch 13, **dadurch gekennzeichnet, dass** die 4,5-Diaminopyrazole der Formel (II) ausgewählt sind unter:
- 4,5-Diamino-1,3-dimethyl-pyrazol,
- 4,5-Diamino-3-methyl-1-phenyl-pyrazol,
- 4,5-Diamino-1-methyl-3-phenyl-pyrazol,
- 4-Amino-1,3-dimethyl-5-hydrazino-pyrazol,
- 1-Benzyl-4,5-diamino-3-methyl-pyrazol,
- 4,5-Diamino-3-*t*-butyl-1-methyl-pyrazol,
- 4,5-Diamino-1-*t*-butyl-3-methyl-pyrazol,
- 4,5-Diamino-1-(β-hydroxyethyl)-3-methyl-pyrazol,
- 4,5-Diamino-1-ethyl-3-methyl-pyrazol,
- 4,5-Diamino-1-ethyl-3-(4'-methoxyphenyl)-pyrazol,
- 4,5-Diamino-1-ethyl-3-hydroxymethyl-pyrazol,
- 4,5-Diamino-3-hydroxymethyl-1-methyl-pyrazol,
- 4,5-Diamino-3-hydroxymethyl-1-isopropyl-pyrazol,
- 4,5-Diamino-3-methyl-1-isopropyl-pyrazol,
- 4-Amino-5-(2'-aminoethyl)-amino-1,3-dimethyl-pyrazol
und deren Additionssalzen mit einer Säure.

19. Zusammensetzung nach Anspruch 14, **dadurch gekennzeichnet, dass** die Triaminopyrazole der Formel (III) unter 3,4,5-Triaminopyrazol, 1-Methyl-3,4,5-triamino-pyrazol, 3,5-Diamino-1-methyl-4-methylamino-pyrazol und 3,5-Diamino-4-(β-hydroxyethyl)-amino-1-methyl-pyrazol und deren Additionssalze mit einer Säure ausgewählt sind.

20. Zusammensetzung nach einem der vorhergehenden Ansprüche 1 bis 19, **dadurch gekennzeichnet, dass** das oder die 4,5- oder 3,4-Diaminopyrazol(e) und/oder das oder die Triaminopyrazol(e) und/ oder das oder die entsprechende(n) Additionssalz(e) mit einer Säure 0,0005 bis 12 Gew.-% des Gesamtgewichts der Farbmittelzusammensetzung ausmachen.

21. Zusammensetzung nach Anspruch 20, **dadurch gekennzeichnet, dass** das oder die 4,5- oder 3,4-Diaminopyrazol(e) und/oder das oder die Triaminopyrazol(e) und/oder das oder die entsprechende(n) Additionssalz(e) mit einer Säure 0,005 bis 6 Gew.-% des Gesamtgewichts der Farbmittelzusammensetzung ausmachen.

22. Zusammensetzung nach einem der Ansprüche 1 bis 21, **dadurch gekennzeichnet, dass** das Gewichtsverhältnis der anorganischen Verbindung(en) und des oder der 4,5- oder 3,4-Diaminopyrazole und/oder des oder der Triaminopyrazole und/oder des oder der Additionssalze mit einer Säure im Bereich von 0,001 bis 100 und vorzugsweise 0,01 bis 10 liegt.

23. Zusammensetzung nach einem der vorhergehenden Ansprüche 1 bis 22, **dadurch gekennzeichnet, dass** die Additionssalze mit einer Säure unter den Hydrochloriden, Hydrobromiden, Sulfaten, Tartraten, Lactaten und Acetaten ausgewählt sind.

24. Zusammensetzung nach einem der vorhergehenden Ansprüche 1 bis 23, **dadurch gekennzeichnet, dass** sie mindestens einen Kuppler enthält.

25. Zusammensetzung nach Anspruch 24, **dadurch gekennzeichnet, dass** der oder die Kuppler etwa 0,0001 bis 10 Gew.-% des Gesamtgewichts der Farbmittelzusammensetzung und noch bevorzugter etwa 0,005 bis 5 Gew.-% des Gesamtgewichts ausmachen.

26. Zusammensetzung nach einem der vorhergehenden Ansprüche 1 bis 25, **dadurch gekennzeichnet, dass** sie mindestens eine zusätzliche Oxidationsbase enthält, die von dem in den Ansprüchen 1 bis 17 definierten Pyrazolen verschieden ist.

27. Zusammensetzung nach Anspruch 26, **dadurch gekennzeichnet, dass** die zusätzliche(n) Oxidationsbase(n) etwa 0,0005 bis 12 Gew.-% des Gesamtgewichts der Farbmittelzusammensetzung ausmachen.

28. Zusammensetzung nach einem der vorhergehenden Ansprüche 1 bis 27, **dadurch gekennzeichnet, dass** sie einen pH-Wert im Bereich von 3 bis 12 aufweist.

29. Zusammensetzung nach einem der vorhergehenden Ansprüche 1 bis 28, **dadurch gekennzeichnet, dass** sie als Flüssigkeit, Creme oder Gel oder beliebigen anderen Formen vorliegt, die geeignet sind, Keratinfasern und insbesondere menschliches Haar zu färben.

30. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 29 aufgetragen wird und die Farbe bei einem sauren, neutralen oder alkalischen pH-Wert mit einem Oxidationsmittel entwickelt wird.

31. Verfahren nach Anspruch 30, **dadurch gekennzeichnet, dass** das Oxidationsmittel, das in der oxidierenden Zusammensetzung vorliegt, unter Wasserstoffperoxid, Hamstoffperoxid, Alkalimetallbromaten, Salzen von Persäuren, wie Perboraten, Percarbonaten und Persulfaten, und Persäuren ausgewählt ist.

32. Verfahren zum Färben von Keratinfasern und insbesondere menschlichen Keratinfasern wie Haaren, **dadurch gekennzeichnet, dass** auf die Fasern in einem ersten Schritt eine Zusammensetzung, die mindestens eine anorganische Verbindung enthält, wie in einem der Ansprüche 1 bis 29 definiert, und in einem .zweiten Schritt eine Zusammensetzung aufgebracht wird, die mindestens ein Diaminopyrazol enthält, wie in einem der Ansprüche 1 bis 29 definiert, wobei nach dem Aufbringen der Zusammensetzung, die die anorganische(n) Verbindung(en) enthält, gegebenenfalls gespült wird, und wobei die Farbe mit einem Oxidationsmittel entwickelt wird.

33. Vorrichtung mit mehreren Abteilungen, die eine erste Abteilung, die eine Farbmittelzusammensetzung nach einem der Ansprüche 1 bis 29 enthält, und eine zweite Abteilung aufweist, die eine oxidierende Zusammensetzung enthält.

34. Vorrichtung mit mehreren Abteilungen, die eine erste Abteilung mit einer anorganischen Verbindung, wie in einem der Ansprüche 1 bis 29 definiert, eine zweite Abteilung mit einem Diaminopyrazol, wie in einem der Ansprüche 1 bis 29 definiert, und eine dritte Abteilung mit einer oxidierenden Zusammensetzung enthält.

## Claims

1. Composition for the oxidation dyeing of human keratin fibres, and in particular of human keratin fibres such as the hair, comprising, in a medium that is suitable for dyeing:
- at least one oxidation base chosen from 4,5- or 3,4-diaminopyrazoles and triaminopyrazoles, and
- at least one mineral compound chosen from silicas, aluminium oxides or hydroxides, aluminium, magnesium and iron hydrosilicates alone or as a mixture, and zinc salts;
the said composition not containing a combination of an oxidation base of the 4,5-diaminopyrazole type, a zinc salt and a derivative of 1,4-diazacycloheptane or a salt thereof, having the following formula: 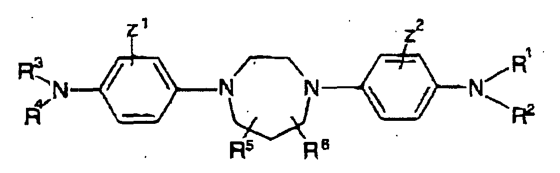 in which R¹, R², R³ and R⁴, which may be identical or different, denote a hydrogen, a C₁-C₄ alkyl or hydroxyalkyl, or a C₂-C₄ dihydroxyalkyl;
Z¹ and Z², which may be identical or different, denote hydrogen, chlorine, fluorine, a C₁-C₄ alkyl, a C₁-C₄ hydroxyalkyl, a C₁-C₄ aminoalkyl, a C₁-C₄ alkoxy, a C₂-C₄ dihydroxyalkyl or an allyl group;
R⁵ and R⁶, which may be identical or different, denote a hydrogen or a C₁-C₄ alkyl.

2. Composition according to Claim 1, **characterized in that** the silica(s) is(are) chosen from crystalline or microcrystalline silicas and non-crystalline silicas.

3. Composition according to Claim 2, **characterized in that** the non-crystalline silica is vitreous silica.

4. Composition according to Claim 2, **characterized in that** the non-crystalline silica is chosen from amorphous silicas such as colloidal silicas, silica gels, precipitated silicas and fumed silicas.

5. Composition according to Claim 1, **characterized in that** the aluminium hydroxides are chosen from the compounds of composition Al(OH)₃ and the compounds of composition AlOOH.

6. Composition according to Claim 1, **characterized in that** the aluminium oxides are chosen from activated aluminas and calcined aluminas.

7. Composition according to Claim 6, **characterized in that** the activated alumina is α alumina.

8. Composition according to Claim 1, **characterized in that** the hydrosilicates are chosen from kaolinite, dicktite, nacrite, halloysite-endellite, pyrophyllite, beidellite, talc, chrysotile, montmorillonite, saponite, nontronite, sauconite, amesite, chamosite and illite.

9. composition according to Claim 1, **characterized in that** the zinc salts are chosen from the salts obtained with a mineral acid.

10. Composition according to Claim 9, **characterized in that** the zinc salts are chosen from zinc chloride, sulphate, nitrate, orthophosphate, ortho-hydrogen phosphate, ortho-dihydrogen phosphate, carbonate and hydrogen carbonate.

11. Composition according to Claim 1, **characterized in that** the zinc salts are chosen from the salts obtained with an organic acid,

12. Composition according to any one of Claims 1 to 11, **characterized in that** the mineral compound(s) represent(s) from 0.00001% to 10% and preferably from 0.001% to 5% by weight approximately relative to the total weight of the dye composition, and even more preferably from 0.001% to 3% by weight approximately relative to this weight.

13. Composition according to any one of Claims 1 to 12, **characterized in that** the 4,5- or 3,4-diaminopyrazoles are chosen from the 4,5- or 3,4-diaminopyrazoles of formula (I) or (II) below, and/or the addition salts thereof with an acid: 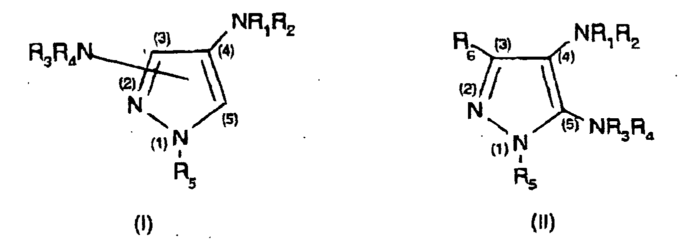 in which:
- R₁, R₂, R₃, R₄ and R₅, which may be identical or different, represent a hydrogen atom; a C₁-C₆ alkyl radical which is unsubstituted or substituted with at least one substituent chosen from OR, NHR, NRR', SR, SOR, SO₂R, COR, COOH, CONH₂, CONHR, CONRR', PO(OH)₂, SH and SO₃X, a non-cationic heterocycle, Cl, Br or I, X denoting a hydrogen atom, Na, K or NH₄, and R and R', which may be identical or different, representing a C₁-C₄ alkyl or alkenyl; a C₂-C₄ hydroxyalkyl radical; a C₂-C₄ aminoalkyl radical; a phenyl radical; a phenyl radical substituted with a halogen atom or a C₁-C₄ alkyl, C₁-C₄ alkoxy, nitro, trifluoromethyl, amino or C₁-C₄ alkylamino radical; a benzyl radical; a benzyl radical substituted with a halogen atom or with a C₁-C₄ alkyl, C₁-C₄ alkoxy, methylenedioxy or amino radical; a radical 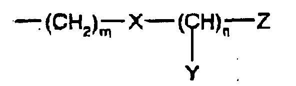
in which m and n are integers, which may be identical or different, between 0 and 3 inclusive, X represents an oxygen atom or an NH group, Y represents a hydrogen atom or a C₁-C₄ alkyl radical, and Z represents a methyl radical when n is equal to 0, or Z represents a C₁-C₄ alkyl radical, a group OR or NR"R'" when n is greater than or equal to 1, R" and R"', which may be identical or different, denoting a hydrogen atom or a C₁-C₄ alkyl radical; or R₅ forms with the nitrogen atom of the group NR₃R₄ in position 5 an at least 4-membered heterocycle,
- R₆ represents a C₁-C₆ alkyl radical; a C₁-C₄ hydroxyalkyl radical; a C₁-C₄ aminoalkyl radical; a (C₁-C₄)-alkylamino (C₁-C₄)alkyl radical; a di(C₁-C₄)alkylamino-(C₁-C₄)alkyl radical; a hydroxy(C₁-C₄)alkylamino(C₁-C₄)-alkyl radical; a (C₁-C₄)alkoxymethyl radical; a phenyl radical; a phenyl radical substituted with a halogen atom or with a C₁-C₄ alkyl, C₁-C₄ alkoxy, nitro, trifluoromethyl, amino or C₁-C₄ alkylamino radical; a benzyl radical; a benzyl radical substituted with a halogen atom or with a C₁-C₄ alkyl, C₁-C₄ alkoxy, nitro, trifluoromethyl, amino or C₁-C₄ alkylamino radical; a heterocycle chosen from thiophene, furan and pyridine, or a radical -(CH₂)ₚ-O-(CH₂)_{q}-OR", in which p and q are integers, which may be identical or different, between 1 and 3 inclusive and R" is as defined above,
it being understood that:
- at least one of the radicals R₁, R₂, R₃ and R₄ represents a hydrogen atom.

14. Composition according to any one of Claims 1 to 12, **characterized in that** the triaminopyrazoles are chosen from the compounds of formula (III) below, and/or the addition salts thereof with an acid: 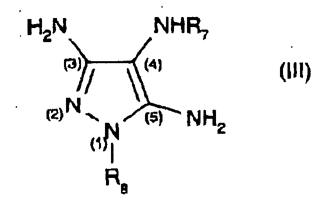 in which:
- R₇ and R₈, which may be identical or different, represent a hydrogen atom or a C₁-C₄ alkyl or C₂-C₄ hydroxyalkyl radical.

15. Composition according to Claim 13, **characterized in that** the 4,5- or 3,4-diaminopyrazoles of formula (I) are chosen from 4,5-diamino-1-(4'-methoxybenzyl)pyrazole, 4,5-diamino-1-(4'-methylbenzyl)pyrazole, 4,5-diamino-1-(4'-chlorobenzyl)-pyrazole, 4,5-diamino-1-(3'-methoxybenzyl)pyrazole, 4-amino-1-(4'-methoxybenzyl)-5-methylaminopyrazole, 4-amino-5-(β-hydroxyethyl)amino-1-(4'-methoxybenzyl)-pyrazole, 4-amino-5-(β-hydroxyethyl)amino-1-methylpyrazole, 4-amino-(3)-5-methylaminopyrazole, 3-(5),4-diaminopyrazole, 4,5-diamino-1-methylpyrazole, 4,5-diamino-1-ethylpyrazole, 4,5-diamino-1-isopropylpyrazole, 4,5-diamino-1-hydroxyethylpyrazole, 4,5-diamino-1-benzylpyrazole, 4-diamino-5-hydroxyethylamino-1-hydroxyethylpyrazole, 4- amino-5-methylamino-1-hydroxyethylpyrazole, 3-amino-4,5,7,8-tetrahydropyrazolo[1,5-a]pyrimidine, 7-amino-2,3-dihydro-1H-imidazole[1,2-b]pyrazole, 3-amino-8-methyl-4,5,7,8-tetrahydropyrazolo[1,5-a]pyrimidine, 2-(4,5-diamino-1-pyrazolyl)-1-ethanesulphonic acid, 2-(4,5-diamino-1-pyrazolyl)acetamide, 2-(4,5-diamino-1-pyrazolyl)acetic acid, 2-(2-dimethylaminoethyl)-2H-pyrazole-3,4-diamine and 2-(2-methoxyethyl)-2H-pyrazole-3,4-diamine, and the addition salts thereof with an acid.

16. Composition according to Claim 15, **characterized in that** the 4,5- or 3,4-diaminopyrazoles of formula (I) are chosen from;
- 4,5-diamino-1-benzylpyrazole,
- 4,5-diamino-1-(4'-chlorobenzyl)pyrazole,
- 4,5-diamino-1-methylpyrazole,
- 4,5-diamino-1-hydroxyethylpyrazole,
- 2-(2-methoxyethyl)-2H-pyrazole-3,4-diamine,
and the addition salts thereof with an acid.

17. Composition according to Claim 13, **characterized in that** the 4,5-diaminopyrazoles of formula (II) are chosen from;
- 1-benzyl-4,5-diamino-3-methylpyrazole,
- 4,5-diamino-1-(β-hydroxyethyl)-3-(4'-methoxyphenyl)-pyrazole,
- 4,5-diamino-1-(β-hydroxyethyl)-3-(4'-methylphenyl)-pyrazole,
- 4,5-diamino-1-(β-hydroxyethyl)-3-(3'-methylphenyl)-pyrazole,
- 4,5-diamino-3-methyl-1-isopropylpyrazole,
- 4,5-diamino-3-(4'-methoxyphenyl)-1-isopropylpyrazole,
- 4,5-diamino-1-ethyl-3-methylpyrazole,
- 4,5-diamino-1-ethyl-3-(4'-methoxyphenyl)pyrazole,
- 4,5-diamino-3-hydroxymethyl-1-methylpyrazole,
- 4,5-diamino-2-ethyl-3-hydroxymethylpyrazole,
- 4,5-diamino-3-hydroxymethyl-1-isopropylpyrazole,
- 4,5-diamino-3-hydroxymethyl-1-tert-butylpyrazole,
- 4,5-diamino-3-hydroxymethyl-1-phenylpyrazole,
- 4,5-diamino-3-hydroxymethyl-1-(2'-methoxyphenyl)-pyrazole,
- 4,5-diamino-3-hydroxymethyl-1-(3'-methoxyphenyl)-pyrazole,
- 4,5-diamino-3-hydroxymethyl-1-(4'-methoxyphenyl)-pyrazole,
- 1-benzyl-4,5-diamino-3-hydroxymethylpyrazole,
- 4,5-diamino-3-methyl-1-(2'-methoxyphenyl)pyrazole,
- 4,5-diamino-3-methyl-1-(3'-methoxyphenyl)pyrazole,
- 4,5-diamino-3-methyl-1-(4'-methoxyphenyl)pyrazole,
- 3-aminomethyl-4,5-diamino-1-methylpyrazole,
- 3-aminomethyl-4,5-diamino-1-ethylpyrazole,
- 3-aminomethyl-4,5-diamino-1-isopropylpyrazole,
- 3-aminomethyl-4,5-diamino-1-tert-butylpyrazole,
- 4,5-diamino-3-dimethylaminomethyl-1-methylpyrazole,
- 4,5-diamino-3-dimethylaminomethyl-1-ethylpyrazole,
- 4,5-diamino-3-dimethylaminomethyl-1-isopropylpyrazole,
- 4,5-diamino-3-dimethylaminomethyl-1-tert-butylpyrazole,
- 4,5-diamino-3-ethylaminomethyl-1-methylpyrazole,
- 4,5-diamino-3-ethylaminomethyl-1-ethylpyrazole,
- 4,5-diamino-3-ethylaminomethyl-1-isopropylpyrazole,
- 4,5-diamino-3-ethylaminomethyl-1-tert-butylpyrazole,
- 4,5-diamino-3-methylaminomethyl-1-methylpyrazole,
- 4,5-diamino-3-methylaminomethyl-1-isopropylpyrazole,
- 4,5-diamino-1-ethyl-3-methylaminomethylpyrazole,
- 1-tert-butyl-4,5-diamino-3-methylaminomethylpyrazole,
- 4,5-diamino-3-[(β-hydroxyethyl)aminomethyl]-1-methylpyrazole,
- 4,5-diamino-3-[(β-hydroxyethyl)aminomethyl]-1-isopropylpyrazole,
- 4,5-diamino-1-ethyl-3-[(β-hydroxyethyl)aminomethyl]-pyrazole,
- 1-tert-butyl-4,5-diamino-3-[(β-hydroxyethyl)aminomethyl]pyrazole,
- 4-amino-5-(β-hydroxyethyl)amino-1,3-dimethylpyrazole,
- 4-amino-5-(β-hydroxyethyl)amino-1-isopropyl-3-methylpyrazole,
- 4-amino-5-(β-hydroxyethyl)amino-1-ethyl-3-methylpyrazole,
- 4-amino-5-(β-hydroxyethyl)amino-1-tert-butyl-3-methylpyrazole,
- 4-amino-5-(β-hydroxyethyl)amino-1-phenyl-3-methylpyrazole,
- 4-amino-5-(β-hydroxyethyl)amino-1-(2-methoxyphenyl)-3-methylpyrazole,
- 4-amino-5-(β-hydroxyethyl)amino-1-(3-methoxyphenyl)-3-methylpyrazole,
- 4-amino-5-(β-hydroxyethyl)amino-1-(4-methoxyphenyl)-3-methylpyrazole,
- 4-amino-5-(β-hydroxyethyl)amino-1-benzyl-3-methylpyrazole,
- 4-amino-1-ethyl-3-methyl-5-methylaminopyrazole,
- 4-amino-1-tert-butyl-3-methyl-5-methylaminopyrazole,
- 4,5-diamino-1,3-dimethylpyrazole,
- 4,5-diamino-3-tert-butyl-1-methylpyrazole,
- 4,5-diamino-1-tert-butyl-3-methylpyrazole,
- 4,5-diamino-1-methyl-3-phenylpyrazole,
- 4,5-diamino-1-(β-hydroxyethyl)-3-methylpyrazole,
- 4,5-diamino-1-(β-hydroxyethyl)-3-phenylpyrazole,
- 4,5-diamino-1-methyl-3-(2'-chlorophenyl)pyrazole,
- 4,5-diamino-1-methyl-3-(4'-chlorophenyl)pyrazole,
- 4,5-diamino-1-methyl-3-(3'-trifluoromethylphenyl)-pyrazole,
- 4,5-diamino-1,3-diphenylpyrazole,
- 4,5-diamino-3-methyl-1-phenylpyrazole,
- 4-amino-1,3-dimethyl-5-phenylaminopyrazole,
- 4-amino-1-ethyl-3-methyl-5-phenylaminopyrazole,
- 4-amino-1,3-dimethyl-5-methylaminopyrazole,
- 4-amino-3-methyl-1-ispropyl-5-methylaminopyrazole,
- 4-amino-3-isobutoxymethyl-1-methyl-5-methylaminopyrazole,
- 4-amino-3-methoxyethoxymethyl-1-methyl-5-methylaminopyrazole,
- 4-amino-3-hydroxymethyl-1-methyl-5-methylaminopyrazole,
- 4-amino-1,3-diphenyl-5-phenylaminopyrazole,
- 4-amino-3-methyl-5-methylamino-1-phenylpyrazole,
- 4-amino-1,3-dimethyl-5-hydrazinopyrazole,
- 5-amino-3-methyl-4-methylamino-1-phenylpyrazole,
- 5-amino-1-methyl-4-(N,N-methylphenyl)amino-3-(4'-chlorophenyl)pyrazole,
- 5-amino-3-ethyl-1-methyl-4-(N,N-methylphenyl)aminopyrazole,
- 5-amino-1-methyl-4-(N,N-methylphenyl)amino-3-phenylpyrazole,
- 5-amino-3-ethyl-4-(N,N-methylphenyl)aminopyrazole,
- 5-amino-4-(N,N-methylphenyl)amino-3-phenylpyrazole,
- 5-amino-4-(N,N-methylphenyl)amino-3-(4'-methylphenyl)-pyrazole,
- 5-amino-3-(4'-chlorophenyl)-4-(N,N-methylphenyl)-aminopyrazole,
- 5-amino-3-(4'-methoxyphenyl)-4-(N,N-methylphenyl)-aminopyrazole,
- 4-amino-5-methylamino-3-phenylpyrazole,
- 4-amino-5-ethylamino-3-phenylpyrazole,
- 4-amino-5-ethylamino-3-(4'-methylphenyl)pyrazole,
- 4-amino-3-phenyl-5-propylaminopyrazole,
- 4-amino-5-butylamino-3-phenylpyrazole,
- 4-amino-3-phenyl-5-phenylaminopyrazole,
- 4-amino-5-benzylamino-3-phenylpyrazole,
- 4-amino-5-(4'-chlorophenyl)amino-3-phenylpyrazole,
- 4-amino-3-(4'-chlorophenyl)-5-phenylaminopyrazole,
- 4-amino-3-(4'-methoxyphenyl)-5-phenylaminopyrazole,
- 1-(4'-chlorobenzyl)-4,5-diamino-3-methylpyrazole,
- 4,5-diamino-3-hydroxymethyl-1-isopropylpyrazole,
- 4-amino-1-ethyl-3-methyl-5-methylaminopyrazole,
- 4-amino-5-(2'-aminoethyl)amino-1,3-dimethylpyrazole,
and the addition salts thereof with an acid.

18. Composition according to Claim 13, **characterized in that** the 4,5-diaminopyrazoles of formula (II) are chosen from:
- 4,5-diamino-1,3-dimethylpyrazole,
- 4,5-diamino-3-methyl-1-phenylpyrazole,
- 4,5-diamino-1-methyl-3-phenylpyrazole,
- 4-amino-1,3-dimethyl-5-hydrazinopyrazole,
- 1-benzyl-4,5-diamino-3-methylpyrazole,
- 4,5-diamino-3-tert-butyl-1-methylpyrazole,
- 4,5-diamino-1-tert-butyl-3-methylpyrazole,
- 4,5-diamino-1-(β-hydroxyethyl)-3-methylpyrazole,
- 4,5-diamino-1-ethyl-3-methylpyrazole,
- 4,5-diamino-1-ethyl-3-(4'-methoxyphenyl)pyrazole,
- 4,5-diamino-1-ethyl-3-hydroxymethylpyrazole,
- 4,5-diamino-3-hydroxymethyl-1-methylpyrazole,
- 4,5-diamino-3-hydroxymethyl-1-isopropylpyrazole,
- 4,5-diamino-3-methyl-1-isopropylpyrazole,
- 4-amino-5-(2'-aminoethyl)amino-1,3-dimethylpyrazole,
and the addition salts thereof with an acid.

19. Composition according to Claim 14, **characterized in that** the triaminopyrazoles of formula (III) are chosen from 3,4,5-triaminopyrazole, 1-methyl-3,4,5-triaminopyrazole, 3,5-diamino-1-methyl-4-methylaminopyrazole and 3,5-diamino-4-(β-hydroxyethyl)amino-1-methylpyrazole, and the addition salts thereof with an acid.

20. Composition according to any one of the preceding Claims 1 to 19, **characterized in that** the 4,5- or 3,4-diaminopyrazole(s) and/or the triaminopyrazole(s) and/or the corresponding addition salt(s) with an acid represent from 0.0005% to 12% by weight relative to the total weight of the dye composition.

21. Composition according to Claim 20, **characterized in that** the 4,5- or 3,4-diaminopyrazole (s) and/or the triaminopyrazole(s) and/or the corresponding addition salt(s) with an acid represent from 0.005% to 6% by weight relative to the total weight of the dye composition.

22. Composition according to any one of the preceding Claims 1 to 21, **characterized in that** the weight ratio of the mineral compound(s) to the 4,5- or 3,4-diaminopyrazole(s) and/or the triaminopyrazole(s) and/or the addition salt(s) with an acid is between 0.001 and 100 and preferably between 0.01 and 10.

23. Composition according to any one of the preceding Claims 1 to 22, **characterized in that** the addition salts with an acid are chosen from the hydrochlorides, hydrobromides, sulphates, tartrates, lactates and acetates.

24. Composition according to any one of the preceding Claims 1 to 23, **characterized in that** it contains at least one coupler.

25. Composition according to Claim 24, **characterized in that** the coupler(s) represent(s) from 0.0001% to 10% by weight approximately relative to the total weight of the dye composition, and even more preferably from 0.005% to 5% by weight approximately relative to this weight.

26. Composition according to any one of the preceding Claims 1 to 25, **characterized in that** it contains at least one additional oxidation base other than the pyrazoles defined in Claims 1 to 17.

27. Composition according to Claim 26, **characterized in that** the additional oxidation base(s) represent(s) from 0.0005% to 12% by weight approximately relative to the total weight of the dye composition.

28. Composition according to any one of the preceding Claims 1 to 27, **characterized in that** it has a pH of between 3 and 12.

29. Composition according to any one of the preceding Claims 1 to 2B, **characterized in that** it is in the form of liquids, creams or gels or in any other form that is suitable for dyeing keratin fibres, and especially human hair.

30. Process for dyeing keratin fibres, and in particular human keratin fibres such as the hair, **characterized in that** at least one dye composition as defined in any one of Claims 1 to 29 is applied to these fibres, and the colour is revealed at acidic, neutral or alkaline pH using an oxidizing agent.

31. Process according to Claim 30, **characterized in that** the oxidizing agent present in the oxidizing composition is chosen from hydrogen peroxide, urea peroxide, alkali metal bromates, persalts such as perborates, percarbonates and persulphates, and peracids.

32. Process for dyeing keratin fibres, and in particular human keratin fibres such as the hair, **characterized in that** a composition containing at least one mineral compound as defined in any one of Claims 1 to 29 is applied to these fibres in a first stage, a composition containing at least one diaminopyrazole as defined in any one of Claims 1 to 29 is applied in a second stage, the application of the composition containing the mineral compound(s) possibly being followed by a rinsing step, the colour being developed using an oxidizing agent.

33. Multi-compartment device comprising a first compartment containing a dye composition as defined in any one of Claims 1 to 29 and a second compartment containing an oxidizing composition.

34. Multi-compartment device comprising a first compartment containing a mineral compound as defined in any one of Claims 1 to 29, a second compartment containing a diaminopyrazole as defined in any one of Claims 1 to 29, and a third compartment containing an oxidizing composition.
